(19)

Europäisches Patentamt

European Patent Office

Office européen des brevets

(11) **EP 4 130 037 A1**

(12) **EUROPEAN PATENT APPLICATION**

(43) Date of publication:
**08.02.2023 Bulletin 2023/06**

(21) Application number: **22182924.5**

(22) Date of filing: **09.01.2018**

(51) International Patent Classification (IPC):
**C07K 16/22** (1995.01)     **C07K 16/28** (1995.01)
**C07K 16/30** (1995.01)

(52) Cooperative Patent Classification (CPC):
**C07K 16/2878; A61P 35/00; C07K 16/22;
C07K 16/2863; C07K 16/2866; C07K 16/2887;
C07K 16/2896; C07K 16/30; C07K 16/3007;
C07K 16/3069;** A61K 2039/505; C07K 2317/31;
C07K 2317/52; C07K 2317/64; C07K 2317/75

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.01.2017   GB 201700345**

(62) Document number(s) of the earlier application(s) in
accordance with Art. 76 EPC:
**18702084.7 / 3 565 838**

(71) Applicant: **F-star Therapeutics Limited
Cambridge, CB22 3AT (GB)**

(72) Inventors:
• **TUNA, Mihriban
Cambridge, CB22 3AT (GB)**
• **HAURUM, John Soerensen
Cambridge, CB22 3AT (GB)**

• **DOODY, Jacqueline Francoise
Cambridge, CB22 3AT (GB)**
• **SUN, Haijun
Cambridge, CB22 3AT (GB)**
• **GASPAR, Miguel
Cambridge, CB22 3AT (GB)**

(74) Representative: **Mewburn Ellis LLP
Aurora Building
Counterslip
Bristol BS1 6BX (GB)**

Remarks:
•This application was filed on 04.07.2022 as a
divisional application to the application mentioned
under INID code 62.
•Claims filed after the date of filing of the application
(Rule 68(4) EPC).

(54) **CONDITIONAL AGONISTS OF IMMUNE RESPONSES**

(57)     The application relates to antibody molecules, which bind to a first and second antigen, the first antigen being a disease antigen, for example a tumour antigen, and the second antigen being a tumour necrosis factor receptor superfamily (TNFRSF) receptor antigen on the surface of an immune cell. The antibody molecules preferably comprise a CDR-based antigen-binding site and an antigen-binding site, which may be located in two or more structural loops of a constant domain of the antibody molecule. The antibody molecules find application, for example, in cancer therapy.

EP 4 130 037 A1

**Description**

<u>Field</u>

The present invention relates to antibody molecules, which bind to a first and second antigen, the first antigen being a disease antigen, for example a tumour antigen, and the second antigen being a tumour necrosis factor receptor superfamily (TNFRSF) receptor antigen on the surface of an immune cell. The antibody molecules preferably comprise a CDR-based antigen-binding site and an antigen-binding site, which may be located in two or more structural loops of a constant domain of the antibody molecule. The antibody molecules of the invention find application, for example, in cancer therapy.

<u>Background</u>

Cell signalling is an essential part of the life of all organisms from bacteria to higher eukaryotes. It normally involves cell surface receptors that interact with soluble or surface expressed ligands. This interaction results in changes to either the receptor or the ligand or both and these changes are then transduced via intracellular proteins that interact with the receptors intracellular domains. The binding of a ligand can induce conformational changes in the receptors causing them to cluster together into dimers or oligomers. This clustering effect then results in activation of the intracellular signalling pathways by multiple mechanisms, such as inducing the kinase activity of the intracellular domains of the receptors, by promoting/inhibiting the interaction between the receptors and their intracellular signalling partners, or by promoting/inhibiting the interaction between the receptors and other cell surface receptors. There are numerous receptor families that are activated in this way such as receptor tyrosine kinases (RTKs, e.g. epidermal growth factor receptor (EGFR)), syndecans, EpH receptors and tumour necrosis factor receptor superfamily (TNFRSF) receptors.

TNFRSF receptors exist as monomers at the cell surface and require clustering into trimers via interaction with their ligands for activation. This clustering results in the activation of the NFkB intracellular signalling pathway. This pathway is initiated by the recruitment of intracellular signalling proteins such as TRAF2, 3, and 5, and the formation of a signalling complex containing IKKα, IKKβ, IKKγ, the p85 subunit of PI3K, and AKT. This signalling complex then induces the phosphorylation and degradation of IκBα, leading to activation of NF-κB1 and entry of p50 and RelA into the nucleus. This activates a transcriptional program that results in proliferation and survival signals. Members of the TNFRSF include several costimulatory proteins with key roles in B and T cell development, survival, immune activation, and antitumour immune responses.

Wajant (2015) Cell Death Differ., 22(1): 1727-41 reports that TNFRSF receptor-specific antibodies typically have no or only a very moderate agonist activity and require secondary crosslinking of antibody-TNFRSF receptor complexes using external crosslinking agents, such as protein A or G or secondary antibodies, or binding of the antibody to plasma membrane localised Fc gamma receptors, in order to induce sufficient receptor activation.

There have been some attempts to generate polypeptides that simultaneously target both a TNFRSF receptor and a cancer cell. For example, an anti-CD137 anticalin protein which is genetically fused to the Her2-specific monoclonal antibody (mAb) trastuzumab has been reported to provide tumour-target-dependent activation of CD137 on lymphocytes at sites where HER2 is overexpressed in a humanised mouse model, but without any improvement in tumour growth inhibition over trastuzumab alone (Hinner et al., "Costimulatory T-cell engagement by PRS-343, a CD137 (4-1BB)/HER2 bispecific, leads to tumour growth inhibition and TIL expansion in humanized mouse model", poster presentation at the CRI-CIMT-AACR International Cancer Immunotherapy Conference: Translating Science into Survival; 2016 Sept 25-28; New York, NY., September 25-26, 2016, New York, NY and PCT publication WO 2016/177802 A1).

<u>Summary</u>

The present inventors have recognised that antibody molecules which include antigen-binding sites in both their variable and constant domains activate TNFRSF receptors conditionally in the presence of a disease antigen, such as a tumour antigen, and are highly effective in activating immune cells in a disease-dependent manner, for example in a tumour microenvironment. This may be useful, for example, in immunotherapy for the treatment of cancer and other diseases.

As described in the background section above, initial ligation of a TNFRSF ligand to its cognate receptor initiates a chain of events that leads to receptor trimerisation, followed by receptor clustering, activation of tumor necrosis factor receptor-associated factors (TRAFs) and subsequent initiation of potent anti-tumour T cell activity. For a therapeutic agent to efficiently achieve activation, several TNFRSF proteins need to be bridged together in a way that mimics a trimeric ligand.

The present inventors have shown that antibody molecules which include antigen-binding sites in their variable and constant domains, wherein one antigen-binding site binds a TNFRSF receptor on the surface of an immune cell and the other a tumour antigen, were capable of inducing clustering and signalling of the TNFRSF receptor when bound to the tumour antigen *in vitro*. The clustering and signalling of the TNFRSF receptor could be achieved regardless of whether the binding site for the TNFRSF receptor was located in the variable or constant domain of the antibody molecule. In other words, the orientation of the two antigen binding sites within the antibody molecule was not important, provided one bound to a TNFRSF receptor and the other to a tumour antigen.

The present inventors have also shown that antibody molecules comprising two antigen-binding sites as detailed above, could induce clustering and signalling of the TNFRSF receptor when the tumour antigen bound by the antibody molecules was a soluble multimeric, e.g. dimeric, molecule.

The present inventors have further shown that antibody molecules comprising two antigen-binding sites as detailed above, were capable of supressing tumour growth *in vivo*. No tumour growth suppression was seen when a combination of two antibody molecules was used where one of the antibody molecules comprised the same constant domain and the other the same variable domain binding site as the bispecific molecule tested by the inventors. This demonstrates that the two binding sites must be present in the same molecule to achieve clustering and signalling of the TNFRSF receptor and thus immune cell activation and corresponding anti-tumour effects.

As mentioned above, one of the advantages of the antibody molecules identified by the present inventors is that they are able to activate TNFRSF receptors conditionally in the presence of a disease antigen, such as a tumour antigen. Specifically, it is thought that binding of the antibody molecules to a multivalent tumour antigen causes clustering of the antibody molecules at the site of the tumour antigen, which in turn leads to clustering and activation of TNFRSF receptors on the immune cell surface. As a consequence, the antibody molecules are likely to have reduced off target effects, as the antibody molecules will show less clustering, if any, outside the tumour microenvironment. The agonistic activity of the antibody molecules is therefore limited to environments in which both the tumour antigen and TNFRSF receptor are present. In other words, the agonistic activity is conditional. In addition, clustering of the antibodies in the presence of the multivalent tumour antigen is thought to assist with clustering of the TNFRSF receptor bound via the second binding site of the antibody molecule, as an increase in the agonistic activity of the antibody molecules was observed when both binding sites of the antibody molecule were bound to their respective targets but not when only one binding site was bound.

The antibody molecules identified by the present inventors include antigen-binding sites in both their variable and constant domains. Binding of both the tumour antigen and the TNFRSF receptor by the antibody molecules is thought to cause bridging between the immune cell expressing the TNFRSF receptor and the tumour antigen in the tumour microenvironment.

In particular, the antibody molecules identified by the present inventors include two antigen-binding sites in their variable and two antigen-binding sites in their constant domains. Thus, both binding sites are bivalent. This has a number of additional advantages. For example, the bivalent binding of both targets is expected to make the bridging between the immune cell expressing the TNFRSF receptor and the tumour antigen in the tumour microenvironment more stable and thereby extend the time during which the immune cell is localised in the tumour microenvironment and can act on the tumour. For example, it is thought that where the constant domain binding sites bind to the tumour antigen bivalently, this stabilised bridging between the tumour antigen and the immune cell, allowing the variable domain binding sites of the antibody molecule to exert their effect by binding to and clustering the TNFRSF receptors on the immune cell surface. Similarly, where the constant domain binding sites bind to the TNFRSF receptors, this is also thought to stabilise bridging between the tumour antigen and the immune cell and was surprisingly shown to lead to clustering and activation of the TNFRSF receptors on the immune cell surface. This was surprising, given the rigid structure and small molecular distance between the constant domains of the antibody molecules (particularly the two CH3 domains) compared to the known flexibility of the antibody molecule in the hinge region, which allows the Fab arms to move and bind to their targets. In light of the tight geometry of the constant domain binding sites, it was not expected that the constant domain binding sites would be able to induce clustering-driven agonism of TNFRSF receptors that may not initially be in close proximity on the cell membrane. However, the results obtained by the present inventors described herein, clearly show that such binding is possible both *in vitro* and *in vivo*. In addition, the fact that both binding sites of the antibody molecules identified by the inventors are bivalent means that either binding site can act to cluster and therefore activate TNFRSF receptors when bound, allowing flexibility in the design and orientation of the two binding sites in the antibody molecule.

The vast majority of known bispecific antibodies are heterodimeric and bind only one target antigen via each Fab arm. This monovalent interaction is frequently insufficient to induce clustering of antigens such as TNFRSF receptors and therefore agonism of immune cells expressing such receptors.

A further feature of the antibody molecules identified by the present inventors is that the two antigen binding sites for the tumour antigen and TNFRSF receptor are both contained within the antibody structure itself. In particular, the antibody molecules do require other proteins to be fused to the antibody molecule via linkers or other means to result in molecule which binds bivalently to both of its targets. This has a number of advantages. Specifically, the antibody molecules identified by the inventors can be produced using methods similar to those employed for the production of standard antibodies, as they do not comprise any additional fused portions. The structure is also expected to result in improved antibody stability, as linkers may degrade over time, resulting in a heterogeneous population of antibody molecules. Those antibodies in the population having only one protein fused will not be able to induce conditional agonism of TNFRSF receptors. Cleavage/degradation of the linker could take place prior to administration or after administration of the therapeutic to the patient (e.g. through enzymatic cleavage or the in vivo pH of the patient), thereby resulting in a reduction of its effectiveness whilst circulating in the patient. As there are no linkers in the antibody molecules identified by the inventors, the antibody molecules are expected to retain the same number of binding sites both before and after administration. Furthermore, the structure of the antibody molecules identified by the inventors is also preferred frrm the perspective of immunogenicity of the molecules, as the introduction of fused proteins or linkers or both may induce immunogenicity when the molecules are administered to a patient, resulting in reduced effectiveness of the therapeutic.

Thus, an aspect of the invention provides an antibody molecule, which binds to a first antigen and a second antigen, the antibody molecule comprising:

(i) a complementarity determining region (CDR)-based antigen-binding site for the first antigen; and
(ii) an antigen-binding site for the second antigen located in a constant domain of the antibody molecule, wherein said antigen-binding site comprises one or more amino acid modifications in one or more structural loops of the constant domain; and

wherein one of the first and second antigens is a disease antigen and the other is a tumour necrosis factor receptor superfamily (TNFRSF) receptor on the surface of an immune cell.

Preferably, the antibody molecule may be a whole antibody, such as an IgG.

Other aspects of the invention provide an isolated nucleic acid encoding an antibody molecule described herein, an expression vector comprising said isolated nucleic acid and a host cell comprising said expression vector.

Another aspect of the invention provides an antibody molecule described herein for use as a medicament.

Other aspects of the invention provide an antibody molecule described herein for use in a method of treating cancer; the use of an antibody molecule described herein in the manufacture of a medicament for use in the treatment of cancer; and a method of treating cancer comprising administering an antibody molecule described herein to an individual in need thereof.

Other aspects and embodiments are described below.

Brief Description of Figures

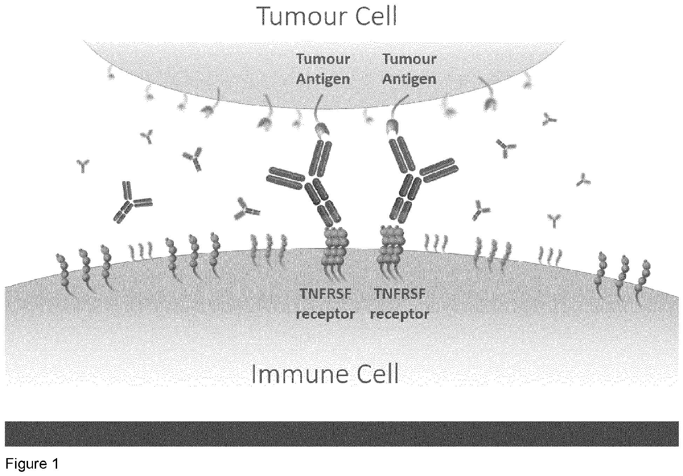

**Figure 1** shows the proposed mechanism of action of the bivalent bispecific antibody molecules of the invention. The illustrated antibody molecule comprises antigen-binding sites at either end of the molecule. The antigen-binding site(s) at one end of the molecule can bind a tumour antigen and the antigen-binding site(s) at the opposite end can bind a TNFRSF receptor on an immune cell. When both ends of the antibody molecule are engaged, as illustrated, this results in crosslinking of multiple receptor molecules in close proximity and, consequently, clustering and activation of the receptor. In Figure 1, the Fc end of the antibody molecule is targeting the TNFRSF receptor and the Fab end is targeting the tumour antigen. However, as demonstrated herein, an antibody molecule in the opposite orientation, where the Fab end targets the TNFRSF receptor and the Fc end targets the tumour antigen, is also able to induce conditional agonism.

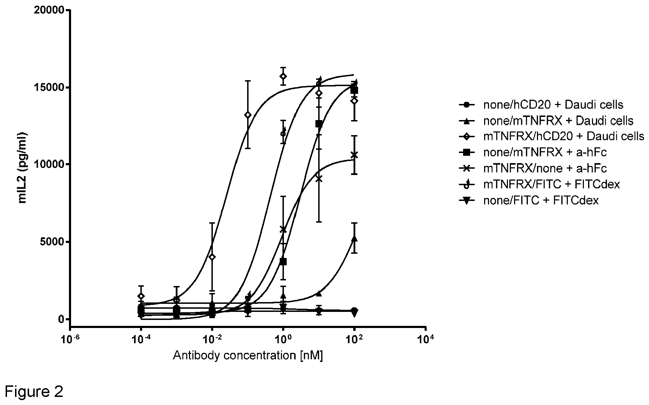

**Figure 2** shows a representative plot of IL-2 release for a T cell activation assay containing hCD20-expressing Daudi cells in the presence of various antibody molecules. Six different antibody molecules were used at increasing concentrations in this assay, labelled according to their Fcab/Fab target (none/FITC, none/hCD20, none/mTNFRX, mTNFRX/none, mTNFRX/FITC and mTNFRX/hCD20). TNFRX is a TNFRSF receptor that is expressed on T cells. Crosslinking agents were used as specified in the figure legend. These were fluorescein isothiocyanate-dextran (FITCdex) and an anti-human IgG Fc antibody (a-hFc). The results show that there is an increase in the activation of T cells when the mTNFRX receptor is targeted and the anti-mTNFRX antibodies are crosslinked, both through the use of crosslinking agents and crosslinking mediated by hCD20 binding. The mTNFRX/hCD20 mAb$^2$ antibody molecule was able to activate T cells in the absence of any crosslinking agent and with a significantly lower $EC_{50}$ compared with the other antibody molecules in the presence of crosslinking agent.

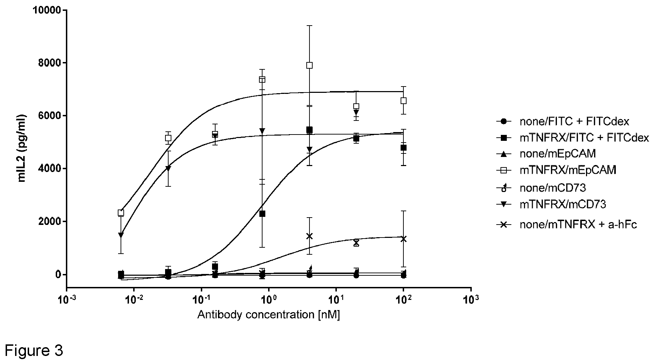

**Figure 3** shows a representative plot of IL-2 release for a T cell activation assay containing EpCAM- and CD73-expressing 4T1 cells in the presence of various antibody molecules. Seven different antibody molecules were used at increasing concentrations in this assay, labelled according to their Fcab/Fab target (none/FITC, mTNFRX/FITC, none/mEpCAM, mTNFRX/mEpCAM, none/mCD73, mTNFRX/mCD73 and none/mTNFRX). FITCdex and a-hFc crosslinking agents were used as specified in the figure legend. The two mAb$^2$ antibody molecules (mTNFRX/mEp-CAM and mTNFRX/mCD73) had the lowest $EC_{50}$ values for activating T cells out of the antibody molecules tested. The results demonstrate that more than one type of cell surface receptor can be used to pair with the anti-TNFRX Fcabs, since CD73 is a GPI-linked cell surface receptor and EpCAM is a transmembrane glycoprotein.

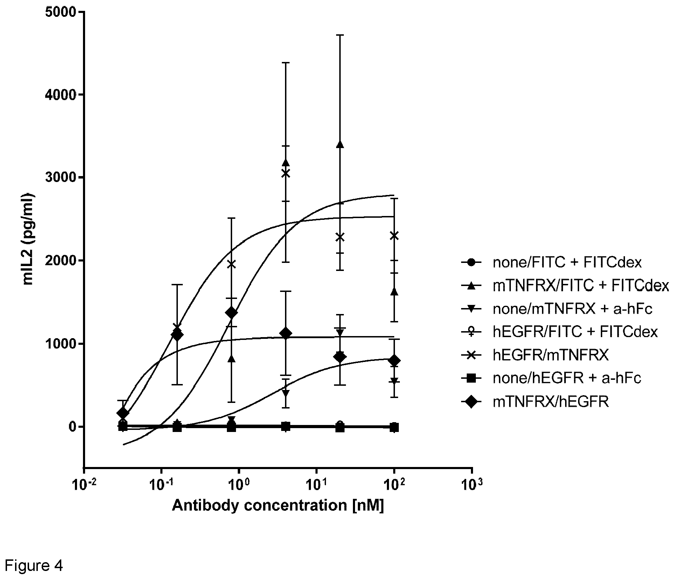

**Figure 4** shows a representative plot of IL-2 release for a T cell activation assay containing hEGFR-expressing CT26 cells in the presence of various antibody molecules. Seven different antibody molecules were used at increasing concentrations in this assay, labelled according to their Fcab/Fab target (none/FITC, mTNFRX/FITC, none/mTNFRX, hEGFR/FITC, hEGFR/mTNFRX, none/hEGFR and mTNFRX/hEGFR). FITCdex and a-hFc crosslinking agents were used as specified in the figure legend. The results show that both orientations of the mAb$^2$ (hEGFR/mTNFRX and mTNFRX/hEGFR) can induce receptor clustering and T cell activation.

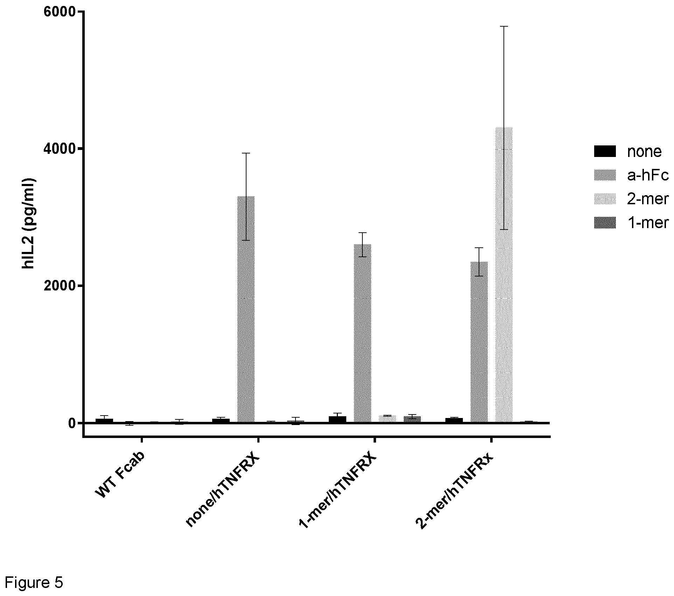

**Figure 5** shows a representative plot of IL-2 release for a T cell activation assay for a single concentration (10 nM) of different antibody molecules. Four different antibody molecules were used in this assay. These molecules were the wild-type Fcab (containing unmodified human IgG1 CH2 and CH3 domains); a hTNFRX mAb (none/hTNFRX); a mAb$^2$ that binds both a soluble monomer (1mer) and hTNFRX (1mer/hTNFRX); and a mAb$^2$ that binds both a soluble dimer (2mer) and hTNFRX (2mer/hTNFRX). The results show that the soluble dimer is able to induce antibody crosslinking of the 2mer/hTNFRX mAb$^2$ but the soluble monomer is unable to induce antibody crosslinking of the 1mer/hTNFRX mAb$^2$ because of the monomeric nature of the antigen. This demonstrates that multimeric but not monomeric soluble factors are capable of inducing antibody crosslinking.

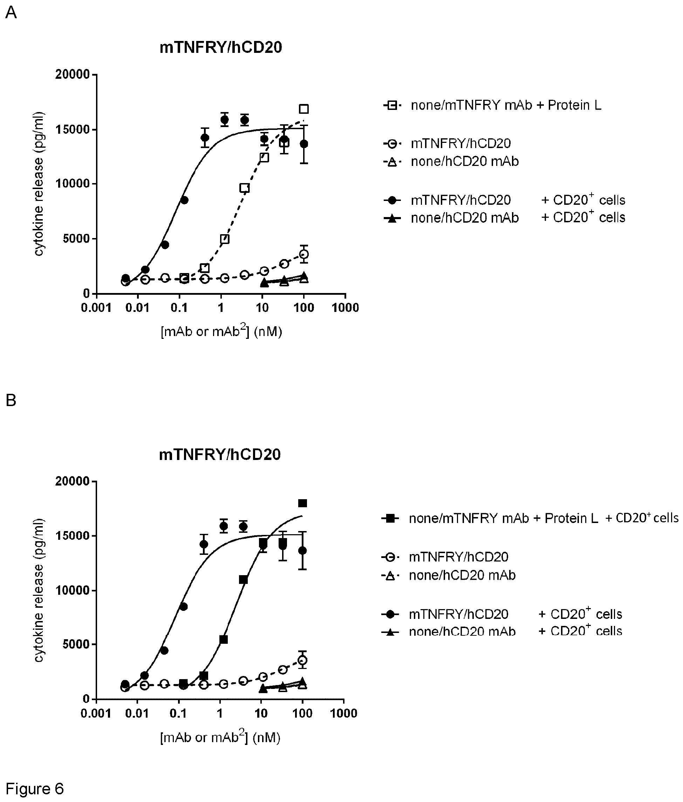

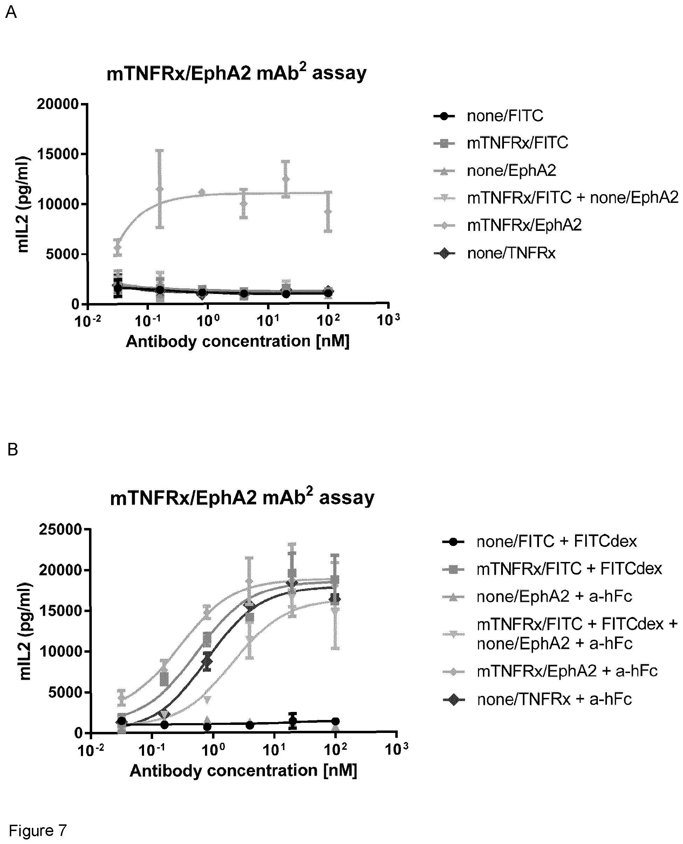

**Figure 6** shows representative plots of IL-2 release for a T cell activation assay containing CD20-expressing Daudi cells in the presence of various antibody molecules. TNFRY is a TNFRSF receptor expressed on T cells. Three different antibody molecules were used at increasing concentrations in this assay, labelled according to their Fcab/Fab target (mTNFRY/hCD20, none/mTNFRY, none/hCD20). Protein L was used as a cross-linking agent for the mTNFRY monoclonal antibody (none/mTNFRY). The antibody molecules were tested in the absence and presence of CD20-expressing Daudi cells (CD20$^+$ cells). Results for the mTNFRY antibody in the absence of CD20$^+$ cells are shown in Figure 6A and in the presence of CD20$^+$ cells in Figure 6B. The results demonstrate that the mAb$^2$ format can be used to induce agonism of a second TNFRSF receptor and that the mTNFRX/hCD20 mAb$^2$ antibody molecule activates T cells with a significantly lower $EC_{50}$ compared with the mTNFRX-targeting mAb antibody molecules. **Figure 7** shows representative plots of IL-2 release for T cell activation assays containing human EphA2-expressing HPAC cells in the presence of various antibody molecules. Six different antibody molecules or combinations were used at increasing concentrations in this assay, labelled according to their Fcab/Fab target (none/FITC, none/EphA2, none/mTNFRX, mTNFRX/FITC, mTNFRX/FITC + none/EphA2 and mTNFRX/EphA2). TNFRX is a TNFRSF receptor that is expressed on T cells. **7A:** The results show that there is an increase in the activation of T cells by the mTNRFX/EphA2 mAb$^2$ antibody in the presence of the EphA2 expressing HPAC cells. This result demonstrates that crosslinking is required for mTNRFX targeting antibodies to increase the activation of T cells and that the mTNRFX/EphA2 mAb$^2$ antibody is the only molecule that can be crosslinked just by the presence of HPAC (EphA2+) cells and does not require any additional non-physiological crosslinking agents. **7B:** The six different antibody molecules or combinations were tested in the presence of crosslinking agents as indicated in the figure key. These were fluorescein isothiocyanate-dextran (FITCdex) and an anti-human IgG Fc antibody (a-hFc). The results show that there is an increase in the activation of T cells when the mTNFRX receptor is targeted and the anti-mTNFRX antibodies are crosslinked. This result demonstrates that crosslinking is required for mTNRFX targeting antibodies to increase the activation of T cells.

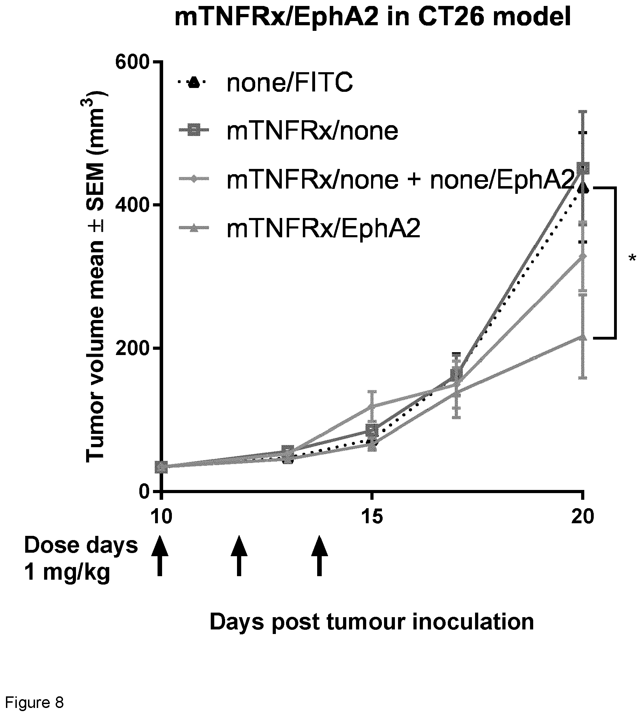

**Figure 8** shows a tumour growth curve of the CT26 syngeneic model in Balb/c mice cohorts. Four different antibody molecules or combinations were dosed at 1mg/kg on days 10, 12 and 14 after tumour inoculation labelled according to their Fcab/Fab target (none/FITC, mTNFRX/none, mTNRFX/none + none/EphA2, mTNFRX/EphA2). The mean tumour volume plus or minus the standard error mean is plotted and the tumour volume on the final day was compared across the different groups using a two-tailed t-test. The mTNFRX/EphA2 mAb$^2$ antibody treated group showed a statistically significant tumour volume reduction as compared to the control antibody none/FITC treated group. This result demonstrates that the mTNRFX/EphA2 mAb$^2$ antibody has a better anti-tumour efficacy *in vivo* against an EphA2 expressing tumour than the combination of the mTNFRX/none and none/EphA2 antibodies indicating that the *in vivo* crosslinking of mTNRFX by the bispecific engagement of mTNRFX and EphA2 mediated by the mTN-RFX/EphA2 mAb$^2$ is effective in controlling tumour growth.

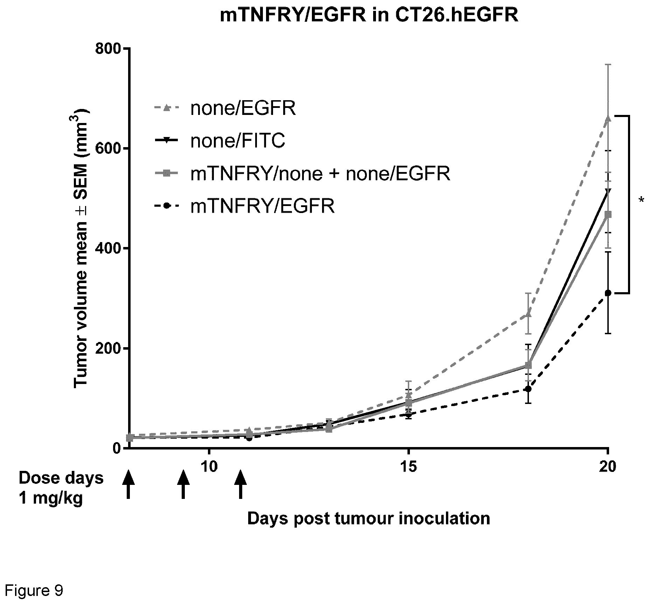

**Figure 9** shows a tumour growth curve of the CT26.hEGFR syngeneic model in Balb/c mice cohorts. Four different antibody molecules or combinations were dosed at 1mg/kg on days 7, 9 and 11 after tumour inoculation labelled according to their Fcab/Fab target (none/FITC, none/EGFR, mTNFRY/none + none/hEGFR and mTNFRY/hEGFR). The mean tumour volume plus or minus the standard error mean is plotted and the tumour volume on the final day was compared across the different groups using a two-tailed t-test. The mTNFRY/hEGFR mAb² antibody treated group showed a statistically significant tumour volume reduction as compared to the control antibody none/EGFR treated group. This result demonstrates that the mTNRFY/hEGFR mAb² antibody has a better anti-tumour efficacy *in vivo* against an EGFR expressing tumour than the combination of the mTNFRX/none and none/hEGFR antibodies indicating that the *in vivo* crosslinking of mTNRFY by the bispecific engagement of mTNRFY and hEGFR mediated by the mTNRFY/hEGFR mAb² is effective in controlling tumour growth.

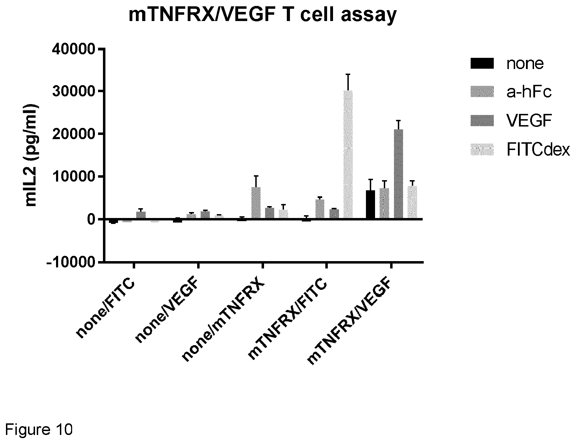

**Figure 10** shows a representative plot of IL-2 release for a T cell activation assay for a single concentration (10 nM) of different antibody molecules. Five different antibody molecules were used in this assay in combination with different crosslinking agents as specified in the figure key. These were fluorescein isothiocyanate-dextran (FITCdex), an anti-human IgG Fc antibody (a-hFc), and VEGF and were used at a 1:1 molar ratio. These molecules were a negative control antibody (none/FITC); a mAb binding VEGF (none/VEGF); a mTNFRX mAb (none/mTNFRX); a mAb² that binds both FITC and mTNFRX (mTNFRX/FITC); and a mAb² that binds both VEGF and mTNFRX (mTNFRX/VEGF). The results show that the mTNRFX antibodies are capable of increasing the activation of T cells only when crosslinked and that the bispecific mAb² antibodies can be crosslinked by either an anti-human IgG Fc antibody or by the Fab target (FITCdex in the case of mTNRFX/FITC and VEGF in the case of mTNRFX/VEGF). The mTNRFX/VEGF mAb² antibody showed a higher background of T cell activation possibly due to the production of VEGF by activated T cells. This demonstrates that VEGF, a soluble factor present at increased levels in the tumour microenvironment, is able to crosslink a mTNRFX/VEGF mAb² antibody and increase T cell activation.

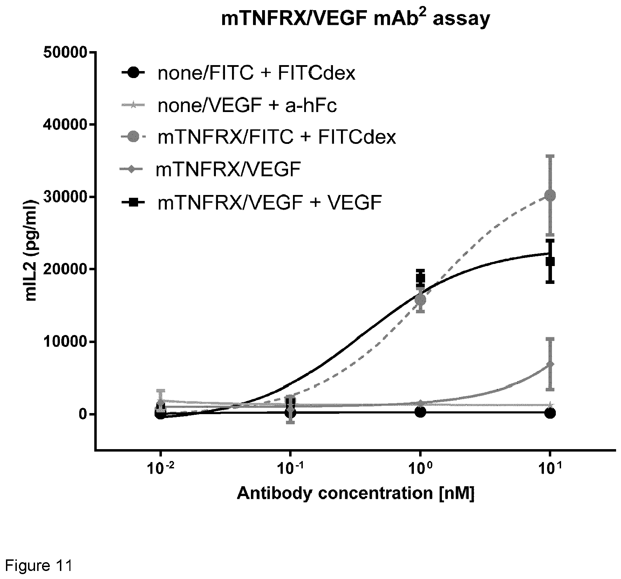

**Figure 11** shows a representative plot of IL-2 release for a T cell activation assay in the presence of various antibody molecules. Four different antibody molecules were used at increasing concentrations in this assay, labelled according to their Fcab/Fab target (none/FITC, none/VEGF, mTNFRX/FITC, mTNFRX/VEGF). TNFRX is a TNFRSF receptor that is expressed on T cells. Crosslinking agents were used as specified in the figure key. These were fluorescein isothiocyanate-dextran (FITCdex), an anti-human IgG Fc antibody (a-hFc) and VEGF. The results show that there is an increase in the activation of T cells when the mTNFRX receptor is targeted and the anti-mTNFRX mAb² antibodies are crosslinked by their Fab targets. This result demonstrates that VEGF is able to crosslink a mTNRFX/VEGF mAb² antibody with comparable efficiency ($EC_{50}$ and maximum response) to an artificial crosslinking agent (FITCdex).

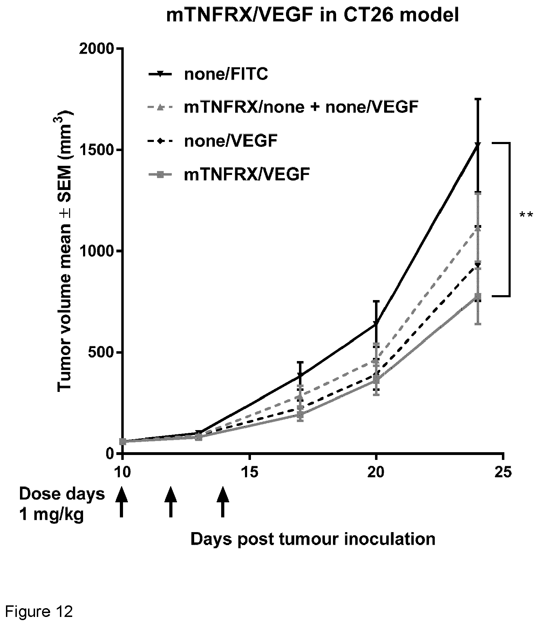

**Figure 12** shows a tumour growth curve of the CT26 syngeneic model in Balb/c mice cohorts. Four different antibody molecules, labelled according to their Fcab/Fab target (none/FITC, none/VEGF, mTNFRX/none + none/VEGF, mTNFRX/VEGF), or combinations thereof were dosed at 1 mg/kg on days 10, 12 and 14 after tumour inoculation. The mean tumour volume plus or minus the standard error mean is plotted and the tumour volume on the final day was compared across the different groups using a two-tailed t-test. The mTNFRX/VEGF mAb² antibody treated group showed a statistically significant tumour volume reduction as compared to the control antibody none/FITC treated group. This result demonstrates that the mTNRFX/VEGF mAb² antibody has a better anti-tumour efficacy *in vivo* against tumour described to have an increased VEGF concentration in its microenvironment than the combination of the mTNRFX/none and none/VEGF antibodies, indicating that the *in vivo* crosslinking of mTNRFX by the bispecific engagement of mTNRFX and VEGF mediated by the mTNRFX/VEGF mAb² is effective in controlling tumour growth.

Detailed Description

This invention relates to antibody molecules, which comprise a complementarity determining region (CDR)-based antigen-binding site for a first antigen and an antigen-binding site for a second antigen in a constant domain of the antibody molecule. One of the first and second antigens is a disease antigen, preferably a tumour antigen, and the other is a tumour necrosis factor receptor superfamily (TNFRSF) receptor on the surface of an immune cell.

Binding of the antibody molecule to the TNFRSF receptor on the immune cell surface in the absence of disease antigen binding does not cause the TNFRSF receptor to form clusters, which stimulate intracellular signalling pathways and activate the immune cell.

Binding of the antibody molecule to both the TNFRSF receptor on the immune cell surface and the disease antigen causes the TNFRSF receptor to form clusters on the immune cell surface, which in turn stimulates intracellular signalling pathways and activates the immune cell. Because the immune cell is activated only in the presence of the disease antigen, the antibody molecule is a conditional agonist which activates the immune cell selectively in the environment of the disease antigen and not elsewhere. For example, the disease antigen may be a tumour antigen and the antibody molecule may activate the immune cell selectively in the microenvironment of a tumour and not elsewhere in a cancer patient.

In addition, the antibody molecules described herein display increased immune cell activation compared to antibody molecules that rely on crosslinking by other mechanisms, such as external crosslinking agents, or crosslinking via Fc gamma receptor interaction. Because the activation of TNFRSF receptors is more efficient, immune cell activation may be achieved at lower concentrations of antibody molecules described herein relative to other antibody molecules. Accordingly, antibody molecules described herein may be able to cause clustering of the TNFRSF receptor in the absence of Fc gamma receptor interaction and/or external crosslinking agents, such as protein A or G or secondary antibodies.

Antibody molecules as described herein are therefore capable of disease-target driven activation of immune cells and, in particular, tumour-target driven activation of immune cells. The activation of immune cells in the environment of a tumour may be useful in immunotherapy, for example for the treatment of cancer.

An antibody molecule as described herein may include any polypeptide comprising a CDR-based antigen-binding site and a constant region antigen binding site. Suitable antibody molecules include immunoglobulins and antigen-binding fragments thereof.

An antibody molecule may be natural or partly or wholly synthetically produced, for example a recombinant molecule.

In preferred embodiments, the antibody molecule is a whole antibody. For example, the antibody molecule may be an IgG, IgA, IgE or IgM, preferably IgG, or any of the isotype subclasses, particularly IgG1, IgG2 and IgG4. For example, the antibody molecule may be a mAb$^2$ (TM) bispecific antibody. A mAb$^2$ bispecific antibody, as referred to herein, is an IgG immunoglobulin which includes a CDR-based antigen binding site in each of its variable regions and one or more antigen binding sites in a constant region, preferably the CH3 domain.

In other embodiments, the antibody molecule may be an antibody fragment that comprises a CDR-based antigen-binding site for the first antigen and a constant domain antigen-binding site for the second antigen. An example of an antibody fragment comprising both CDR sequences and CH3 domain is a minibody, which comprises an scFv joined to a CH3 domain (Hu et al. (1996), Cancer Res., 56(13):3055-61). Unless the context requires otherwise, the term "antibody molecule", as used herein, is thus equivalent to "antibody molecule or fragment thereof". Preferred antibody fragments may comprise more than one CDR-based antigen-binding site and/or constant domain i.e. they may be multivalent.

An antibody molecule may be monoclonal.

An antibody molecule may be chimeric, humanised or human. Chimeric molecules comprising an immunoglobulin binding domain, or equivalent, fused to another polypeptide are therefore included. Cloning and expression of chimeric antibodies are described in EP-A-0120694 and EP-A-0125023

An antibody molecule as described herein may be isolated, in the sense of being free from contaminants, such as antibodies able to bind other polypeptides and/or serum components.

Antibodies and methods for their construction and use are well-known in the art and are described in, for example, Holliger & Hudson, Nature Biotechnology 23(9):1126-1136 (2005). It is possible to take monoclonal and other antibodies and use techniques of recombinant DNA technology to produce other antibodies or chimeric molecules which retain the specificity of the original antibody. Such techniques may involve introducing CDRs, variable regions, and/or the constant domain sequences providing the second antigen-binding site, into a different immunoglobulin. Introduction of the CDRs of one immunoglobulin into another immunoglobulin is described, for example, in EP-A-184187, GB 2188638A and EP-A-239400. Similar techniques may be employed for the relevant constant domain sequences. Alternatively, a hybridoma or other cell producing an antibody molecule may be subject to genetic mutation or other changes, which may or may not alter the binding specificity of antibodies produced.

An antibody molecule described herein may be modified to reduce or abrogate binding to Fc receptors and/or complement. This may be useful in reducing the antibody-dependent cell-mediated cytotoxicity (ADCC) and/or the complement-dependent cytotoxicity (CDC) activity of the antibody molecule. For example, the CH2 domain of an antibody molecule may comprise a mutation to reduce or abrogate binding of the CH2 domain to one or more Fc γ receptors, such as FcγRI, FcγRIIa, FcγRIIb, FcγRIII and/or to complement. Mutations that reduce or abrogate binding of the CH2 domain to one or more Fc γ receptors and complement are known and include the "LALA mutation" described in Bruhns, et al., Blood 113(16):3716-25 (2009) and Xu et al., Cellular Immunology 200(1):16-26 (2000). Thus, the antibody molecule may comprise a CH2 domain, wherein the CH2 domain comprises alanine residues at positions 1.3 and 1.2 of the CH2 domain, wherein the numbering is according to the IMGT numbering scheme.

In some embodiments, an antibody molecule described herein may lack an Fc γ receptor binding site and may display no binding or substantially no binding to an Fc γ receptor. In addition, or alternatively, the antibody molecule described herein may lack a C1q binding site and may display no binding or substantially no binding to C1q. Mutations for abrogating binding to C1q are known in the art.

An antibody molecule may further comprise one or more amino acid substitutions, deletions or insertions which improve one or more properties of the antibody, for example kinetics such as affinity, on and off-rates and/or other biophysical characteristics such as functional half-life.

An antibody molecule described herein comprises one or more antigen binding sites, which binds to a disease antigen, such as a tumour antigen, and one or more antigen binding sites which bind to a TNFRSF receptor. Binding in this context may refer to specific binding. The term "specific" may refer to the situation in which the antibody molecule will not show any significant binding to molecules other than its specific binding partner(s). The term "specific" is also applicable where the antibody molecule is specific for particular epitopes, such as epitopes on a tumour antigen and a TNFRSF receptor, that are carried by a number of antigens, in which case the antibody molecule will be able to bind to the various antigens carrying the epitope.

An antibody molecule of the invention comprises one or more CDR-based antigen-binding sites for the first antigen.

A CDR-based antigen-binding site is an antigen-binding site in an antibody variable region. A CDR-based antigen-binding site, which is formed by six CDRs; three light chain variable domain (VL) CDRs and three heavy chain variable domain (VH) CDRs. The contributions of the different CDRs to the binding of the antigen may vary in different antigen binding sites. The preparation of antibody molecules against a given antigen, such as a tumour antigen or TNFRSF receptor, and determination of the CDR sequences of such antibody molecules, is well established and many suitable techniques are known in the art.

A CDR-based antigen-binding site may comprise the HCDR3 of an antibody which binds to a TNFRSF receptor or a disease antigen, such as a tumour antigen. The HCDR3 is known to play a role in determining the specificity of an antibody molecule (Segal et al., (1974), PNAS, 71:4298-4302; Amit et al., (1986), Science, 233:747-753; Chothia et al., (1987), J. Mol. Biol., 196:901-917; Chothia et al., (1989), Nature, 342:877-883; Caton et al., (1990), J. Immunol., 144:1965-1968; Sharon et al., (1990a), PNAS, 87:4814-4817; Sharon et al., (1990b), J. Immunol., 144:4863-4869; Kabat et al., (1991b), J. Immunol., 147:1709-1719). The CDR-based antigen-binding site may further comprise the HCDR1, HCDR2, LCDR1, LCDR2 and/or LCDR3 of the antibody which binds to the TNFRSF receptor or disease antigen, such as tumour antigen.

The sequences of the CDRs may be readily determined from the VH and VL domain sequences of any given antibody using routine techniques. The CDR sequences may, for example, be determined according to Kabat (Kabat, E.A et al (1991). Sequences of Proteins of Immunological Interest, 5th edit., NIH Publication no. 91-3242. U.S.Department of Health and Human Services) or the international ImMunoGeneTics information system (IMGT: Lefranc, M.-P. et al. Nucleic Acids Res. 43, D413-22 (2015)).

The three VH domain CDRs of the antigen-binding site may be located within an immunoglobulin VH domain and the three VL domain CDRs may be located within an immunoglobulin VL domain. For example, the CDR-based antigen-binding site may be located in an antibody variable region.

The antibody molecule may have one or preferably more than one, for example two, CDR-based antigen binding sites for the first antigen. The antibody molecule thus may comprise one VH and one VL domain but preferably comprises two VH and two VL domains, i.e two VH/VL domain pairs, as is the case in naturally-occurring IgG molecules.

In some preferred embodiments, the antibody molecule may be an IgG molecule comprising two variable regions, each variable region comprising a CDR-based antigen binding site for the first antigen.

An antibody molecule of the invention comprises an antigen-binding site for the second antigen that is located in a constant region (i.e. a constant region antigen-binding site).

The antigen-binding site for the second antigen may be located in any constant region of the antibody molecule. For example, the antigen-binding site for the second antigen may be located in one or more of the CH4, CH3, CH2, CH1 or CL domains, preferably the CH3 or CH2 domain, most preferably the CH3 domain.

The antigen binding site may be composed of one or more, for example one, two, three or more, structural loops of the constant domain of the antibody molecule.

The structural loops of an antibody constant domain include the AB, BC, CD, DE, EF, and FG structural loops. The antigen binding site may comprise two or more of the AB, BC, CD, DE, EF, and FG structural loops of the constant domain, most preferably the AB, CD and/or the EF structural loops.

The positions of the structural loops in antibody constant domains are well-known in the art. For example, the structural loops of the CH3 domain are located at residues 7-21 (AB loop), 25-39 (BC loop), 41-81 (CD loop), 83-85 (DE loop), 89-103 (EF loop) and 106-117 (FG loop) of the CH3 domain, wherein the residues are numbered according to IMGT (ImMunoGeneTics™) numbering scheme. The locations of the structural loop positions in other constant domains may be easily determined.

The structural loops of the constant domain may comprise one or more amino acid modifications in order to form the antigen-binding site for the second antigen. One or more amino acid modifications may include amino acid substitutions, additions, or deletions. The introduction of amino acid modifications into the structural loop regions of antibody constant domains to create antigen-binding sites for target antigens is well-known in the art and is described, for example, Wozniak-Knopp G et al. (2010) Protein Eng Des. 23 (4): 289-297; WO2006/072620 and WO2009/132876.

Examples of constant domain binding sites are provided below.

In a preferred embodiment, the antibody molecules of the invention comprises one or more amino acid modifications (substitution, addition, and/or deletion) in the AB, CD and/or EF structural loops, located at positions 7-21, 41-81, and/or 89-103 of the CH3 domain, respectively. More preferably, the antibody molecules of the invention comprise one or more amino acid modifications (substitution, addition, and/or deletion) at positions 11-18, 43-78 and/or 92-101 of the CH3 domain. Most preferably, the antibody molecules of the invention comprise one or more amino acid modifications (substitution, addition, and/or deletion) at positions 12-18, 45.1 to 78, 92 to 94, and/or 97-98 of the CH3 domain to provide an antigen-binding site for a first antigen as set out herein. The unmodified CH3 domain preferably comprises or consists of the sequence set forth in SEQ ID NO: 3. The residue numbering is according to IMGT numbering scheme.

The antibody molecule may comprise one, two or more than two constant domain antigen-binding sites for the second antigen.

An antibody Fc region containing a constant domain antigen-binding site for the second antigen may be referred to as an Fcab. In some embodiments, an antibody molecule as described herein may be produced by providing an antibody which binds the first antigen and replacing its Fc region with an Fcab which binds the second antigen. An antibody molecule as described herein may also be produced by providing an antibody which binds the first antigen and replacing its CH3 domains with CH3 domains which bind the second antigen or alternatively, replacing fragments of the CH3 domains with CH3 domain fragments which bind to the second antigen,

In a preferred embodiment, the antibody is an antibody which binds to a first antigen and a second antigen, the antibody molecule comprising:

(i) a CDR-based antigen-binding site for the first antigen formed by an immunoglobulin VH domain and an immunoglobulin VL domain; and
(ii) an antigen-binding site for the second antigen located in a CH3 domain of the antibody molecule, wherein said antigen-binding site comprises one or more amino acid modifications in one or more structural loops of the CH3 domain; and

wherein one of the first and second antigens is a tumour antigen and the other is a TNFRSF receptor on the surface of an immune cell.

In a further preferred embodiment, the antibody is an antibody which binds to a first antigen and a second antigen, the antibody molecule comprising:

(i) two CDR-based antigen-binding sites for the first antigen, each formed by an immunoglobulin VH domain and an immunoglobulin VL domain; and
(ii) two antigen-binding sites for the second antigen located in the two CH3 domains of the antibody molecule, wherein said antigen-binding site comprises one or more amino acid modifications in one or more structural loops of the CH3 domain; and

wherein one of the first and second antigens is a tumour antigen and the other is a TNFRSF receptor on the surface of an immune cell.

In a yet further preferred embodiment, the antibody is a complete immunoglobulin molecule, e.g. a complete IgG1 molecule, which binds to a first antigen and a second antigen, the immunoglobulin molecule comprising:

(i) two CDR-based antigen-binding sites for the first antigen, each formed by an immunoglobulin VH domain and an immunoglobulin VL domain; and
(ii) two antigen-binding sites for the second antigen located in the two CH3 domains of the immunoglobulin molecule, wherein said antigen-binding site comprises one or more amino acid modifications in one or more structural loops of the CH3 domain; and

wherein one of the first and second antigens is a tumour antigen and the other is a TNFRSF receptor on the surface of an immune cell, and
wherein the immunoglobulin molecule further comprises CH1, CH2 and CL domains.

Bispecific molecules, in which a second antigen binding site is provided via conjugation of a second antigen-binding moiety to an antibody or fragment thereof, usually via a linker, are known in the art. Such molecules have a potential disadvantage in that linker cleavage may result in loss of the second antigen-binding site. In the context of the present invention, loss of one of the two binding sites would render the antibody molecule of the invention inactive. Thus, the antibody molecule of the invention preferably does not comprise a second antigen-binding moiety (such as an anticalin, single domain antibody (dAb), single variable domain (sVD), single-chain variable fragment (scFv), or antigen-binding fragment (Fab)) conjugated e.g. to the C-terminus of the antibody molecule, for example via a linker sequence.

An antibody molecule described herein binds to the first antigen through a CDR-based antigen binding site and the second antigen through a constant domain antigen binding site.

In some embodiments, the first antigen may be a TNFRSF receptor on the surface of an immune cell, and the second antigen may be a disease antigen, such as a tumour antigen.

In other embodiments, the first antigen may be a disease antigen, such as a tumour antigen, and the second antigen may be a TNFRSF receptor.

An antibody molecule may have a dissociation constant for the first and/or second antigen of less than 50 nM, less than 40 nM, less than 30 nM, less than 20 nM, less than 10 nM, or less than 1 nM. For example, an antibody molecule may have an affinity for the first and/or second antigen of 0.1 to 50 nM, e.g. 0.5 to 10 nM. Binding kinetics and affinity (expressed as the equilibrium dissociation constant, $K_d$) of the antibody molecule may be determined using standard techniques, such as surface plasmon resonance e.g. using BIAcore analysis.

Tumour necrosis factor receptor superfamily (TNFRSF) receptors are membrane-bound cytokine receptors that comprise an extracellular cysteine rich domain which binds one or more ligands of the tumour necrosis factor superfamily (TNFSF).

The TNFRSF receptor may be located on the surface of an immune cell. Upon binding of a TNFRSF ligand, TNFRSF receptors form clusters on the immune cell surface which activate the immune cell. For example, ligand bound TNFRSF receptors may form multimers, such as trimers, or clusters of multimers. The presence of clusters of ligand-bound TNFRSF receptors stimulates intracellular signalling pathways which activate the immune cell.

By engaging both the TNFRSF receptor on the cell surface and a tumour antigen, the antibody molecules described herein cause the TNFRSF receptors to cluster and activate the immune cell.

TNFRSF receptors include CD27, CD40, EDA2R, EDAR, FAS, LTBR, RELT, TNFRSF1A, TNFRSF1B, TNFRSF4, TNFRSF6B, TNFRSF8, TNFRSF9, TNFRSF10A-10D, TNFRSF11A, TNFRSF11B, TNFRSF12A, TNFRSF13B, TNFRSF13C, TNFRSF14, TNFRSF17, TNFRSF18, TNFRSF19, TNFRSF21 and TNFRSF25.

CD27 (TNFRSF7: Gene ID 939) has the reference amino acid sequence of NP_001233.1 and may be encoded by the reference nucleotide sequence of NM_001242.4. CD40 (TNFRSF5: Gene ID 958) has the reference amino acid sequence of NP_001241.1 and may be encoded by the reference nucleotide sequence of NM_001250.5. EDA2R (TNFRSF27: Gene ID 60401) has the reference amino acid sequence of NP_001186616.1 and may be encoded by the reference nucleotide sequence of NM_001199687.2. EDAR (Gene ID 10913) has the reference amino acid sequence of NP_071731.1 and may be encoded by the reference nucleotide sequence of NM_022336, 3. FAS (TNFRSF6: Gene ID 355) has the reference amino acid sequence of NP_000034.1 and may be encoded by the reference nucleotide sequence of NM_000043.5. LTBR (TNFRSF3: Gene ID 4055) has the reference amino acid sequence of NP_001257916.1 and may be encoded by the reference nucleotide sequence of NM_001270987.1. RELT (TNFRSF19L: Gene ID 84957) has the reference amino acid sequence of NP_116260.2 and may be encoded by the reference nucleotide sequence of NM_032871.3. TNFRSF1A (Gene ID 7132) has the reference amino acid sequence of NP_001056.1 and may be encoded by the reference nucleotide sequence of NM_001065.3. TNFRSF1B (Gene ID 7133) has the reference amino acid sequence of NP_001057.1 and may be encoded by the reference nucleotide sequence of NM_001066.2. TNFRSF4 (Gene ID 7293) has the reference amino acid sequence of NP_003318 and may be encoded by the reference nucleotide sequence of NM_003327). TNFRSF6B (Gene ID 8771) has the reference amino acid sequence of NP_003814.1 and may be encoded by the reference nucleotide sequence of NM_003823.3. TNFRSF8 (Gene ID 943) has the reference amino acid sequence of NP_001234.3 and may be encoded by the reference nucleotide sequence of NM_001243.4. TNFRSF9 (Gene ID 3604) has the reference amino acid sequence of NP_001552 and may be encoded by the reference nucleotide sequence of NM001561). TNFRSF10A (Gene ID 8797) has the reference amino acid sequence of NP_003835.3 and may be encoded by the reference nucleotide sequence of NM_003844.3. TNFRSF10B (Gene ID 8795) has the reference amino acid sequence of NP_003833.4 and may be encoded by the reference nucleotide sequence of NM_003842.4. TNFRSF10C (Gene ID 8794) has the reference amino acid sequence of NP_003832.2 and may be encoded by the reference nucleotide sequence of NM_003841.4. TNFRSF10D (Gene ID 8793) has the reference amino acid sequence of NP_003831.2 and may be encoded by the reference nucleotide sequence of NM_003840.4. TNFRSF11A (Gene ID 8792) has the reference amino acid sequence of XP_011524547.1 and may be encoded by the reference nucleotide sequence of XM_11526245.2. TNFRSF11B (Gene ID 4982) has the reference amino acid sequence of NP_002537.3 and may be encoded by the reference nucleotide sequence of NM_002546.3. TNFRSF12A (Gene ID 51330) has the reference amino acid sequence of NP_057723.1 and may be encoded by the reference nucleotide sequence of NM_016639.2. TNFRSF13B (Gene ID 23495) has the reference amino acid sequence of NP_0036584.1 and may be encoded by the reference nucleotide sequence of NM_012452.2. TNFRSF13C (Gene ID 115650) has the reference amino acid sequence of NP_443177.1 and may be encoded by the reference nucleotide sequence of NM_052945.3. TNFRSF14 (Gene ID 8764) has the reference amino acid sequence of NP_001284534.1 and may be encoded by the reference nucleotide sequence of NM_001297605.1. TNFRSF17 (Gene ID 608) has the reference amino acid sequence of NP_001183.2 and may be encoded by the reference nucleotide sequence of NM_001192.2. TNFRSF18 (Gene ID 8784) has the reference amino acid sequence of NP_004195.2 and may be encoded by the reference nucleotide sequence of NM_004186,1. TNFRSF19 (Gene ID 55504) has the reference amino acid sequence of NP_001191387.1 and may be encoded by the reference nucleotide sequence of NM_001204458.1. NFRSF21 (Gene ID 27242) has the reference amino acid sequence of NP_055267.1 and may be encoded by the reference nucleotide sequence of NM_014452.4. TNFRSF25 (DR3: Gene ID 8718) binds to ligand TNFSF15 (TL1A) has the reference amino acid sequence of NP_001034753.1 and may be encoded by the reference nucleotide sequence of NM_001039664.1.

In some embodiments, the TNFRSF receptor is not nerve growth factor receptor (NGFR).

In some embodiments, the TNFRSF receptor is not CD40, TNFRSF4, TNFRSF9, CD27, or GITR. For example, the TNFRSF receptor may be selected from the group consisting of CD27, EDA2R, EDAR, FAS, LTBR, RELT, TNFRSF1A, TNFRSF1B, TNFRSF6B, TNFRSF8, TNFRSF10A-10D, TNFRSF11A, TNFRSF11B, TNFRSF12A, TNFRSF13B, TNFRSF13C, TNFRSF14, TNFRSF17, TNFRSF18, TNFRSF19, TNFRSF21 and TNFRSF25.

The immune cell, which expresses the TNFRSF receptor on its surface, may be a T cell, an antigen presenting cell (APC), an NK cell and/or a B cell, preferably a T cell.

Following activation by an antibody molecule as described herein in the vicinity of a disease antigen, the activated immune cell may initiate, promote or take part in an immune response, for example an immune response against a pathogen or a cancer cell. An overview of the role the immune system plays in recognizing and eradicating cancer cells is provided by Chen and Mellman, Immunity 2013 39(1):1-10.

A disease antigen is an antigen that is present at a site of disease but is not present elsewhere in an individual. Disease antigens may include pathogenic antigens and tumour antigens.

Pathogenic antigens may include antigens from viruses or bacteria associated with infectious disease. Activation of immune cells as described herein in the vicinity of pathogenic antigens may be useful in the treatment of infectious disease.

Preferably, the disease antigen is a tumour antigen. A tumour antigen is an antigen that is predominantly present in the environment of a tumour, and is not ubiquitously present elsewhere in an individual. For example, the tumour antigen may be present on the surface of tumour cells or may be present on other stromal cells of the tumour microenvironment or in biological fluids in the vicinity of a tumour. The tumour antigen is therefore a marker of the location of tumour cells in an individual.

In some embodiments, the tumour antigen may be an antigen that is located on the surface of a cancer cell. Preferably, the tumour antigen is upregulated or overexpressed on tumour cells, whereas it is not abundantly expressed by the corresponding normal somatic cells from the same tissue in the absence of a tumour.

In some embodiments, the tumour antigen is upregulated or overexpressed on stromal cells of the tumour microenvironment, compared with stromal cells of the corresponding normal tissue in the absence of a tumour.

Preferred tumour antigens exist on the cell surface and are not rapidly internalised.

Tumour antigens that are suitable for targeting by the antibody molecules of the invention may be identified using methods that are known in the art. For example, an antibody molecule targeting a TNFRSF receptor and a tumour antigen can be used in an assay where a TNFRSF receptor expressing cell is co-cultured with a tumour antigen expressing cell and activation of the TNFRSF receptor expressing cell is measured, for example by ELISA, a proliferation assay or cytotoxicity assay.

A cell surface tumour antigen may be a Tumour-Associated Antigen (TAA) or a Tumour-specific antigen (TSA).

Tumour antigens expressed by cancer cells may include, for example, cancer-testis (CT) antigens encoded by cancer-germ line genes, such as MAGE-A1, MAGE-A2, MAGE- A3, MAGE-A4, MAGE-A5, MAGE-A6, MAGE-A7, MAGE-A8, MAGE-A9, MAGE-A10, MAGE-A11, MAGE-A12, GAGE-I, GAGE-2, GAGE-3, GAGE-4, GAGE-5, GAGE-6, GAGE-7, GAGE-8, BAGE-I, RAGE- 1, LB33/MUM-1, PRAME, NAG, MAGE-Xp2 (MAGE-B2), MAGE-Xp3 (MAGE-B3), MAGE-Xp4 (MAGE-B4), MAGE- C1/CT7, MAGE-C2, NY-ESO-I, LAGE-I, SSX-I, SSX-2(HOM-MEL-40), SSX-3, SSX-4, SSX-5, SCP-I and XAGE and immunogenic fragments or variants thereof (Simpson et al. Nature Rev (2005) 5, 615-625, Gure et al., Clin Cancer Res (2005) 11, 8055-8062; Velazquez et al., Cancer Immun (2007) 7, 1 1 ; Andrade et al., Cancer Immun (2008) 8, 2; Tinguely et al., Cancer Science (2008); Napoletano et al., Am J of Obstet Gyn (2008) 198, 99 e91-97).

Other cell surface tumour antigens include, for example, AFP, $\alpha_v\beta_3$ (vitronectin receptor), $\alpha_v\beta_6$, CA125 (MUC16), CD4, CD20, CD22, CD33, CD52, CD56, CD66e, CD80, CD140b, CD227 (MUC1), EGFR (HER1), EpCAM, GD3 ganglioside, HER2, prostate-specific membrane antigen (PSMA), prostate specific antigen (PSA), CD5, CD19, CD21, CD25, CD37, CD30, CD33, CD45, HLA-DR, anti-idiotype, carcinoembyronic antigen (CEA), e.g. carcinoembryonic antigen-related cell adhesion molecule 5 (CEACAM5), TAG-72, Folate-binding protein, A33, G250, ferritin, glycolipids such as gangliosides, carbohydrates such as CA-125, IL-2 receptor, fibroblast activation protein (FAP), IGF1R, B7H3, PDL1, CD200, EphA2, and mesothelin or variants thereof. These and other cell surface tumour antigens are described in Carter et al., Endocr Relat Cancer 11(4): 659-87 (2004), Scott, A. M. and Renner, C. (2001). Tumour Antigens Recognized by Antibodies. eLS and Cheever et al., Clin Cancer Res (2009) 15(17): 5323-37.

Other tumour antigens include out-of-frame peptide-MHC complexes generated by the non-AUG translation initiation mechanisms employed by "stressed" cancer cells (Malarkannan et al. Immunity 1999 Jun; 10(6):681-90).

Other tumour antigens include peptide-MHC complexes on the surface of tumour cells or of cells of the tumour microenvironment, where the peptide-MHC complexes comprise a tumour-specific neoantigen peptide fragment of a mutated intracellular tumour antigen, and where the peptide neoantigen harbours one or more tumour-specific mutations (Gubin et al J Clin Invest. (2015) 125(9): 3413-21). Other tumour antigens are well-known in the art (see for example WO00/20581; Cancer Vaccines and Immunotherapy (2000) Eds Stern, Beverley and Carroll, Cambridge University Press, Cambridge) The sequences of these tumour antigens are readily available from public databases but are also found in WO 1992/020356 A1, WO 1994/005304 A1, WO 1994/023031 A1, WO 1995/020974 A1, WO 1995/023874 A1 and WO 1996/026214 A1.

Preferred tumour antigens include HER2, FAP, EpCAM, CEACAM5, CD20, CD73, PSMA, mesothelin, EphA2, IGF1R, CD200, $\alpha_V\beta_6$, PDL1, B7H3 and EGFR.

HER2 (ERBB2; Gene ID 2064) may have the reference amino acid sequence of NP_001005862.1 and may be encoded by the reference nucleotide sequence of NM_001005862.2. FAP (Gene ID 2191) may have the reference amino acid sequence of NP_001278736.1 and may be encoded by the reference nucleotide sequence of NM_001291807.1. EpCAM (Gene ID 4072) may have the reference amino acid sequence of NP_002345.2 and may be encoded by the reference nucleotide sequence of NM_002354.2. CEACAM5 (Gene ID 1048) may have the reference amino acid sequence of NP_001278413.1and may be encoded by the reference nucleotide sequence of NM_001291484.2. CD20 (MS4A1; Gene ID 931) may have the reference amino acid sequence of NP_068769.2 and may be encoded by the reference nucleotide sequence of NM_021950.3. CD73 (NT5E; Gene ID 4907) may have the reference amino acid sequence of NP_001191742.1 and may be encoded by the reference nucleotide sequence of NM_001204813.1. PSMA (FOLH1; Gene ID 2346) may have the reference amino acid sequence of NP_001014986.1 and may be encoded by the reference nucleotide sequence of NM_001014986.1. Mesothelin (MSLN; Gene ID 10232) may have the reference amino acid sequence of NP_001170826.1 and may be encoded by the reference nucleotide sequence of NM_001177355.2. EphA2 (Gene ID 1969) may have the reference amino acid sequence of NP_001316019.1 and may be encoded by the reference nucleotide sequence of NM_001329090.1. IGF1R (Gene ID 3480) may have the reference amino acid sequence of NP_000866.1 and may be encoded by the reference nucleotide sequence of NM_000875.4. CD200 (Gene ID 4345) may have the reference amino acid sequence of NP_001004196.2 and may be encoded by the reference nucleotide sequence of NM_001004196.3. $\alpha_V\beta_6$ is a heterodimer composed of the integrin subunit alpha V and integrin subunit beta 6. Integrin subunit alpha V (ITGAV; Gene ID 3685) may have the reference amino acid sequence of NP_001138471.1 and may be encoded by the reference nucleotide sequence of NM_001144999.2. Integrin subunit beta 6 (ITGB6; Gene ID 3694) may have the reference amino acid sequence of NP_000879.2 and may be encoded by the reference nucleotide sequence of NM_000888.4. PDL1 (CD274; Gene ID 29126) may have the reference amino acid sequence of NP_001254635.1 and may be encoded by the reference nucleotide sequence of NM_001267706.1. B7H3 (CD276; Gene ID 80381) may have the reference amino acid sequence of NP_001019907.1 and may be encoded by the reference nucleotide sequence of NM_001024736.1. EGFR (Gene ID 1956) may have the reference amino acid sequence of NP_001333826.1 and may be encoded by the reference nucleotide sequence of NM_001346897.1.

In other embodiments, the tumour antigen may be a soluble tumour antigen, for example a growth factor that is produced by or in response to cancer cells. A soluble factor may be upregulated or overexpressed in biological fluids in the vicinity of a tumour. A soluble tumour antigen may be multimeric, for example a dimer or a trimer. A soluble tumour antigen may be present in higher concentrations at the tumour site or in the tumour microenvironment than elsewhere in the body of a patient. The tumour microenvironment and associated soluble tumour antigens are described in more detail in Bhome et al., Front Cell Dev Biol. 2015 3:33.

Suitable soluble tumour antigens include VEGF, HGF and SDF1.

VEGF (VEGFA; gene ID 7422) has the reference amino acid sequence of NP_001020537.2 and may be encoded by the reference nucleotide sequence of NM_001025366.2. HGF (gene ID 3082) has the reference amino acid sequence of NP_000592.3 and may be encoded by the reference nucleotide sequence of NM_000601.5. SDF1 (CXCL12; gene ID 6387) has the reference amino acid sequence of NP_000600.1 and may be encoded by the reference nucleotide sequence of NM_000609.6.

Another aspect of the invention provides an isolated nucleic acid encoding an antibody molecule described above. The skilled person would have no difficulty in preparing such nucleic acids using methods well-known in the art. An isolated nucleic acid may be used to produce an antibody molecule described above, for example, by expression in a bacterial, yeast, insect or mammalian host cell. A preferred host cell is a mammalian cell such as a CHO, HEK or NS0 cell. The nucleic acid will generally be provided in the form of a recombinant vector for expression.

Another aspect of the invention provides a vector comprising the nucleic acid described above.

Suitable vectors can be chosen or constructed, containing appropriate regulatory sequences, including promoter sequences, terminator fragments, polyadenylation sequences, enhancer sequences, marker genes and other sequences as appropriate. Preferably, the vector contains appropriate regulatory sequences to drive the expression of the nucleic acid in mammalian cells. A vector may also comprise sequences, such as origins of replication, promoter regions and selectable markers, which allow for its selection, expression and replication in bacterial hosts such as *E. coli.*

Vectors may be plasmids, viral e.g. phage, or phagemid, as appropriate. For further details see, for example, Molecular Cloning: a Laboratory Manual: 3rd edition, Russell et al., 2001, Cold Spring Harbor Laboratory Press. Many known techniques and protocols for manipulation of nucleic acid, for example in preparation of nucleic acid constructs, mutagenesis, sequencing, introduction of DNA into cells and gene expression, are described in detail in Current Protocols in Molecular Biology, Ausubel et al. eds. John Wiley & Sons, 1992.

A nucleic acid or vector as described herein may be introduced into a host cell.

Another aspect of the invention provides a recombinant cell comprising a nucleic acid or vector that expresses a polypeptide comprising or consisting of an antibody molecule as described above.

A range of host cells suitable for the production of recombinant antibody molecules are known in the art. Suitable host cells may include prokaryotic cells, in particular bacteria such as *Escherichia coli* and *Lactococcus lactis* and eukaryotic cells, including mammalian cells such as CHO and CHO-derived cell lines (Lec cells), HeLa, COS, HEK293 and HEK-EBNA cells, amphibian cells such as Xenopus oocytes, insect cells such as *Trichoplusia ni,* Sf9 and Sf21 and yeast cells, such as *Pichia pastoris.*

Techniques for the introduction of nucleic acid into cells are well established in the art and any suitable technique may be employed, in accordance with the particular circumstances. For eukaryotic cells, suitable techniques may include calcium phosphate transfection, DEAE-Dextran, electroporation, liposome-mediated transfection and transduction using retrovirus or other virus, e.g. adenovirus, AAV, lentivirus or vaccinia. For bacterial cells, suitable techniques may include calcium chloride transformation, electroporation and transfection using bacteriophage.

Marker genes such as antibiotic resistance or sensitivity genes may be used in identifying clones containing nucleic acid of interest, as is well-known in the art.

The introduced nucleic acid may be on an extra-chromosomal vector within the cell or the nucleic acid may be integrated into the genome of the host cell. Integration may be promoted by inclusion of sequences within the nucleic acid or vector which promote recombination with the genome, in accordance with standard techniques.

A method may further comprise isolating and/or purifying the antibody molecule. This may be achieved using any convenient method known in the art. Techniques for the purification of recombinant antibody molecules are well-known in the art and include, for example HPLC, FPLC or affinity chromatography, e.g. using Protein A or Protein L. In some embodiments, purification may be performed using an affinity tag on the polypeptide as described above.

Another aspect of the invention provides a method of producing an antibody molecule described above comprising expressing a nucleic acid encoding an antibody molecule described above in a host cell and optionally isolating and/or purifying the antibody molecule thus produced.

Antibody molecules produced as described may be investigated further, for example the pharmacological properties and/or activity may be determined. Methods and means of protein analysis are well-known in the art.

Antibody molecules as described herein may be useful for therapeutic applications. For example, an antibody molecule as described herein may be administered to an individual for the treatment of cancer.

Whilst an antibody molecule may be administered alone, antibody molecules will usually be administered in the form of a pharmaceutical composition, which may comprise at least one component in addition to the antibody molecule. A method may comprise formulating the antibody molecule into a pharmaceutical composition, optionally with at least one component, for example a pharmaceutically acceptable excipient or other substance as described below.

Another aspect of the invention provides a pharmaceutical composition comprising an antibody molecule described above and a pharmaceutically acceptable excipient.

Pharmaceutical compositions may comprise, in addition to the antibody molecule, a pharmaceutically acceptable excipient, carrier, buffer, stabilizer or other materials well known to those skilled in the art. The term "pharmaceutically acceptable" as used herein pertains to compounds, materials, compositions, and/or dosage forms which are, within the scope of sound medical judgement, suitable for use in contact with the tissues of a subject (e.g., human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. The precise nature of the carrier or other material will depend on the route of administration, which may be by infusion, injection or any other suitable route, as discussed below.

For parenteral, for example sub-cutaneous or intra-venous administration, e.g. by injection, the pharmaceutical composition comprising the antibody molecule may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or nonionic surfactants, such as TWEEN™, PLURONICS™ or polyethylene glycol (PEG).

In some embodiments, antibody molecules may be provided in a lyophilised form for reconstitution prior to administration. For example, lyophilised antibody molecules may be re-constituted in sterile water and mixed with saline prior to administration to an individual.

An antibody molecule as described herein may be used in a method of treatment of the human or animal body, including prophylactic or preventative treatment. A method of treatment may comprise administering an antibody molecule to an individual in need thereof. Related aspects of the invention provide;

(i) an antibody molecule described above for use as a medicament,
(ii) an antibody molecule described above for use in a method of treating cancer in a patient,
(iii) the use of an antibody molecule described above in the manufacture of a medicament for use in a method of treating cancer in a patient; and,
(iv) a method of treating cancer in an individual, wherein the method comprises administering to the individual a therapeutically effective amount of an antibody molecule described above.

Cancer may be characterised by the abnormal proliferation of malignant cancer cells and may include leukaemias, such as AML, CML, ALL and CLL, lymphomas, such as Hodgkin lymphoma, non-Hodgkin lymphoma and multiple myeloma, and solid cancers such as sarcomas, skin cancer, melanoma, bladder cancer, brain cancer, breast cancer, uterus cancer, ovary cancer, prostate cancer, lung cancer, colorectal cancer, cervical cancer, liver cancer, head and neck cancer, oesophageal cancer, pancreas cancer, renal cancer, adrenal cancer, stomach cancer, testicular cancer, cancer of the gall bladder and biliary tracts, thyroid cancer, thymus cancer, cancer of bone, and cerebral cancer.

Cancer cells within an individual may be immunologically distinct from normal somatic cells in the individual (i.e. the cancerous tumour may be immunogenic). For example, the cancer cells may be capable of eliciting a systemic immune response in the individual against one or more antigens expressed by the cancer cells. The antigens that elicit the immune response may be tumour antigens or may be shared by normal cells.

An individual suitable for treatment as described above may be a mammal, such as a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orang-utan, gibbon), or a human.

In some preferred embodiments, the individual is a human. In other preferred embodiments, non-human mammals, especially mammals that are conventionally used as models for demonstrating therapeutic efficacy in humans (*e.g.* murine, primate, porcine, canine, or rabbit animals) may be employed.

In some embodiments, the individual may have minimal residual disease (MRD) after an initial cancer treatment.

An individual with cancer may display at least one identifiable sign, symptom, or laboratory finding that is sufficient to make a diagnosis of cancer in accordance with clinical standards known in the art. Examples of such clinical standards can be found in textbooks of medicine such as Harrison's Principles of Internal Medicine, 15th Ed., Fauci AS et al., eds., McGraw-Hill, New York, 2001. In some instances, a diagnosis of a cancer in an individual may include identification of a particular cell type (e.g. a cancer cell) in a sample of a body fluid or tissue obtained from the individual.

Treatment may be any treatment and therapy, whether of a human or an animal (e.g. in veterinary applications), in which some desired therapeutic effect is achieved, for example, the inhibition or delay of the progress of the condition, and includes a reduction in the rate of progress, a halt in the rate of progress, amelioration of the condition, cure or remission (whether partial or total) of the condition, preventing, ameliorating, delaying, abating or arresting one or more symptoms and/or signs of the condition or prolonging survival of a subject or patient beyond that expected in the absence of treatment.

Treatment as a prophylactic measure (i.e. prophylaxis) is also included. For example, an individual susceptible to or at risk of the occurrence or re-occurrence of cancer may be treated as described herein. Such treatment may prevent or delay the occurrence or re-occurrence of cancer in the individual.

In particular, treatment may include inhibiting cancer growth, including complete cancer remission, and/or inhibiting cancer metastasis. Cancer growth generally refers to any one of a number of indices that indicate change within the cancer to a more developed form. Thus, indices for measuring an inhibition of cancer growth include a decrease in cancer cell survival, a decrease in tumour volume or morphology (for example, as determined using computed tomographic (CT), sonography, or other imaging method), a delayed tumour growth, a destruction of tumour vasculature, improved performance in delayed hypersensitivity skin test, an increase in the activity of anti-cancer immune cells or other anti-cancer immune responses, and a decrease in levels of tumour-specific antigens. Activating or enhancing immune responses to cancerous tumours in an individual may improve the capacity of the individual to resist cancer growth, in particular growth of a cancer already present the subject and/or decrease the propensity for cancer growth in the individual.

Administration may be in a "therapeutically effective amount", this being sufficient to show benefit to a patient. The actual amount administered, and rate and time-course of administration, will depend on the nature and severity of what is being treated, the particular patient being treated, the clinical condition of the individual patient, the cause of the disorder, the site of delivery of the composition, the type of antibody molecule, the method of administration, the scheduling of administration and other factors known to medical practitioners. Prescription of treatment, e.g. decisions on dosage etc., is within the responsibility of general practitioners and other medical doctors, and may depend on the severity of the symptoms and/or progression of a disease being treated. Appropriate doses of antibody molecules are well known in the art (Ledermann et al. (1991) Int. J. Cancer 47: 659-664; and Bagshawe et al. (1991) Antibody, Immunoconjugates and Radiopharmaceuticals 4: 915-922). Specific dosages indicated herein, or in the Physician's Desk Reference (2003) as appropriate for an antibody molecule being administered, may be used. A therapeutically effective amount or suitable dose of an antibody molecule can be determined by comparing *in vitro* activity and *in vivo* activity in an animal model. Methods for extrapolation of effective dosages in mice and other test animals to humans are known. The precise dose will depend upon a number of factors, including whether the size and location of the area to be treated, and the precise nature of the antibody molecule. Treatments may be repeated at daily, twice-weekly, weekly or monthly intervals, at the discretion of the physician.

A typical antibody dose will be in the range 100 $\mu$g to 1 g for systemic applications, and 1 $\mu$g to 1 mg for topical applications. An initial higher loading dose, followed by one or more lower doses, may be administered. Typically, the antibody will be a whole antibody, e.g. the IgG1 or IgG4 isotype. This is a dose for a single treatment of an adult patient, which may be proportionally adjusted for children and infants, and also adjusted for other antibody formats in proportion to molecular weight. Treatments may be repeated at daily, twice-weekly, weekly or monthly intervals, at the discretion of the physician. The treatment schedule for an individual may be dependent on the pharmocokinetic and pharmacodynamic properties of the antibody composition, the route of administration and the nature of the condition being treated.

Treatment may be periodic, and the period between administrations may be about two weeks or more, e.g. about three weeks or more, about four weeks or more, about once a month or more, about five weeks or more, or about six weeks or more. For example, treatment may be every two to four weeks or every four to eight weeks. Suitable formulations and routes of administration are described above.

The method may further comprise administering at least one additional therapeutic agent to the patient.

An additional therapeutic agent may include a chemotherapeutic agent, anti-tumour vaccine, radionuclide, immunotherapeutic agent, such as a therapeutic antibody molecule or therapeutic fragment thereof, cytokine, oncolytic viruses, CAR-T cells, or agent for hormone therapy

A chemotherapeutic agent may be selected from the group consisting of: taxanes, cyctotoxic antibiotics, tyrosine kinase inhibitors, PARP inhibitors, B_RAF enzyme inhibitors, alkylating agents, platinum analogs, nucleoside analogs, thalidomide derivatives, antineoplastic chemotherapeutic agents and others. Taxanes include docetaxel, paclitaxel and nabpaclitaxel; cytotoxic antibiotics include actinomycin, bleomycin, anthracyclines, doxorubicin and valrubicin; tyrosine kinase inhibitors include erlotinib, gefitinib, axitinib, PLX3397, imatinib, cobemitinib and trametinib; PARP inhibitors include piraparib; B-Raf enzyme inhibitors include vemurafenib and dabrafenib; alkylating agents include dacarbazine, cyclophosphamide, temozolomide; platinum analogs include carboplatin, cisplatin and oxaliplatin; nucleoside analogs include gemcitabine and azacitidine; antineoplastics include fludarabine. Other chemotherapeutic agents suitable for use in the present invention include methotrexate, defactinib, entinostat, pemetrexed, capecitabine, eribulin, irinotecan, fluorouracil, and vinblastine.

An additional therapeutic agent may include an anti-tumour vaccine. Vaccination strategies for the treatment of cancers has been both implemented in the clinic and discussed in detail within scientific literature (such as Rosenberg, S. 2000 Development of Cancer Vaccines). This mainly involves strategies to prompt the immune system to respond to various cellular markers expressed by autologous or allogenic cancer cells by using those cells as a vaccination method, both with or without granulocyte-macrophage colony-stimulating factor (GM-CSF). GM-CSF provokes a strong response in antigen presentation and works particularly well when employed with said strategies.

Other aspects and embodiments of the invention provide the aspects and embodiments described above with the term "comprising" replaced by the term "consisting of' and the aspects and embodiments described above with the term "comprising" replaced by the term "consisting essentially of".

It is to be understood that the application discloses all combinations of any of the above aspects and embodiments described above with each other, unless the context demands otherwise. Similarly, the application discloses all combinations of the preferred and/or optional features either singly or together with any of the other aspects, unless the context demands otherwise.

Modifications of the above embodiments, further embodiments and modifications thereof will be apparent to the skilled person on reading this disclosure, and as such, these are within the scope of the present invention.

All documents and sequence database entries mentioned in this specification are incorporated herein by reference in their entirety for all purposes.

"and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein.

Certain aspects and embodiments of the invention will now be illustrated by way of example and with reference to the figures described above.

Experiments

Example 1 - Preparation of Fcabs and mAb²s

1.1 Selection of Fcab molecules

*1.1.1 Naive selection of mTNFRX Fcab clones*

Libraries expressing Fcab clones on the yeast cell surface were incubated with a biotinylated recombinant murine TNF receptor (mTNFRX) fused to a murine IgG Fc domain (mTNFRX-mFc) and sorted by MACS using streptavidin coated beads. Three rounds of FACS selections were then performed using decreasing concentrations of biotinylated mTNFRX-mFc in the presence of a fivefold molar excess of mFc. The cells were stained with streptavidin-allophycocyanin (APC) (BD Bioscience, 349024) or anti-Biotin-APC (Miltenyi Biotec, 130-090-856) and sorted using a FACSAria (BD Bioscience). Individual clones from enriched populations were screened for antigen binding and positive binders were subcloned and expressed as soluble Fcabs (containing a truncated hinge) in HEK Expi293 cells (Fcabs cloned into pTT5 vector [National Research Council of Canada] transfected using ExpiFectamine 293 Transfection kit [ThermoFisher Scientific, A14524] into Expi293F cells [ThermoFisher Scientific, A14527]). Fcabs for further maturation were identified by screening for cell binding.

A "wild-type" Fcab comprises the CH2 and CH3 domain sequences of human IgG1. This is set forth both with and without a LALA mutation in SEQ ID NO: 1 and SEQ ID NO: 2, respectively. The Fcab clones selected for these experiments contained mutations within the AB loop (residues 7-21), the CD loop (residues 41-81) and the EF loop (residues 89-103) of the CH3 domain. All residues are numbered according to the IMGT numbering scheme.

*1.1.2 Affinity maturation of TNFRX Fcabs*

Affinity maturation libraries were constructed by randomising seven residues in the AB loop (residues 12 - 18), six residues in the CD loop (residues 45.1-78) and five residues in the EF loop (92-94 and 97-98) using an equimolar distribution of amino acids excluding cysteine.

The affinity maturation libraries were generated in phage and three rounds of selections were performed against biotinylated mTNFRX-mFc alternating between streptavidin-coated (ThermoFisher Scientific, 11205D) and neutravidin-coated (ThermoFisher Scientific, 14203 and A2666) Dynabeads. Decreasing antigen concentrations from 50 nM to 10 nM and 1 nM were used to identify high affinity binders. Round three outputs were screened by phage ELISA and positive binders were subcloned and expressed as described above.

26 CD and 36 EF loops identified in the affinity maturation were shuffled and expressed as soluble Fcabs (containing a truncated hinge) in HEK Expi293 cells as described above. HEK supernatants containing the Fcabs were screened for improved off-rates by measuring binding to biotinylated mTNFRX-mFc using Dip and Read™ Streptavidin Biosensors (Pall FortéBio, 18-5050) on an Octet QKᵉ System (Pall FortéBio) and for cell binding as described in Example 1.3.

*1.1.3 Naive selection of mTNFRY Fcab clone*

Libraries expressing Fcab clones on the yeast cell surface were incubated with a biotinylated recombinant murine TNF receptor (mTNFRY) fused to a murine IgG Fc domain (mTNFRY-mFc) and sorted by MACS using streptavidin coated beads. FACS selections were then performed using decreasing concentrations of biotinylated mTNFRY-mFc in the presence of a fivefold molar excess of mFc. The cells were stained with streptavidin-allophycocyanin (APC) (BD Bioscience, 349024) or anti-Biotin-APC (Miltenyi Biotec, 130-090-856) and sorted using a FACSAria (BD Bioscience). Individual clones from enriched populations were screened for antigen binding and positive binders were subcloned and expressed as soluble mock mAb² (mTNFRY/HeID1.3).

A "wild-type" Fcab comprises the CH2 and CH3 domain sequences of human IgG1. This is set forth both with and without a LALA mutation in SEQ ID NO: 1 and SEQ ID NO: 2, respectively. The Fcab clones selected for these experiments contained mutations within the AB loop (residues 7-21) and the EF loop (residues 89-103) of the CH3 domain. All residues are numbered according to the IMGT numbering scheme.

The anti-mTNFRY Fcab clone referred to in 1.2 below and selected for use in the construction of the mTNFRY/hCD20 and mTNFRY/hEGFR mAb[2] antibodies identified in Examples 5 and 7 below was a naïve clone which did not undergo any affinity maturation.

1.2 Construction and expression of mAb[2]

An anti-mouse TNFRX Fcab and an anti-mouse TNFRY Fcab were identified and selected from the experimental procedures described in 1.1 above for use in the preparation of various mAb[2] in order to allow the characterisation of these Fcabs in mAb[2] format. These mAb[2] were prepared from the selected anti-mouse TNFRX and anti-mouse TNFRY Fcabs and the variable regions of the antibodies are detailed in Table 1.

The nomenclature system used for the mAb[2] is Fcab/Fab, where Fcab describes the binding target of the Fc modified region (Fcab) and Fab describes the binding target of the Fab arms of the mAb[2]. In both cases, where "m" precedes the target name this indicates binding to the murine version of the target and where "h" precedes the target name this indicates binding to the human version of the target. Where neither "m" nor "h" precedes the target name, this indicates binding to both the murine and human versions of the target. Where G1 is used for the Fcab this indicates the unchanged human IgG1 Fc region (WT). The LALA mutation is located in the CH2 domain of the heavy chain and is known to reduce binding Fc gamma receptor binding (Bruhns, P., *et al.* (2009); and Xu, D. *et al.* (2000)). The mAb[2] were produced by transient expression in HEK293-6E cells and purified using mAb Select SuRe protein A columns.

The affinity of mTNFRX/hEGFR for mTNFRX-mFc was determined using a Biacore T200. 5000 RU of EGFR-Fc (R&D Systems, 344-ER) was immobilised on a Series S CM5 chip (GE Healthcare, BR100530). 5 nM mTNFRX/hEGFR was injected at 10 $\mu$l/min for 1 min followed by mTNFRX-mFc at various concentration at 70 $\mu$l/min for 3 min and buffer injection for 10 min. The chip was regenerated by injection of 30 mM NaOH at 30 $\mu$l/min for 10 s. The data was double reference subtracted and analysed using BIAevaluation 3.2 software to calculate kinetic constants.

The affinity of mTNFRY/HeID1.3 for its mTNFRY target was determined using a similar Biacore method as that used to determine the affinity of mTNFRX/hEGFR for its mTNFRX target.

1.3 Binding affinity of Fcab to mouse TNFRX expressed on cells as determined by flow cytometry

Lentiviral transduction methodology was used to generate DO11.10 cells (National Jewish Health) over-expressing mouse TNFRX using the Lenti-X HTX Packaging System (Clontech, Cat. No 631249). Lenti-X expression vector (pLVX) (Clontech, Cat. No 631253), containing the mouse TNFRX cDNA, was co-transfected with a Lenti-X HTX Packaging Mix into the Lenti-X 293T Cell Line (Clontech, Cat. No 632180) to generate virus. The DO11.10 cell line was transduced using the lentiviral vectors produced with the Lenti-X HTX Packaging System.

The affinity of the anti-mouse TNFRX Fcab and mAb[2] to cells expressing mouse TNFRX (DO11.10 cell line transfected with mouse TNFRX) was measured using flow cytometry. mAb[2] and control mAb dilutions (2 $\times$ final concentration) were prepared in triplicate in 1 $\times$ DPBS (Gibco, 14190-094). DO11.10-mTNFRX cell suspensions were prepared in PBS+2%BSA (Sigma, A7906) and seeded at 4 $\times$ 10$^{-6}$ cell/ml with 50 $\mu$l/well in V-bottomed 96-well plates (Costar, 3897). 50$\mu$l of the mAb[2] or control mAb (none/mTNFRX) dilutions were added to the wells containing cells (final volume 100 $\mu$l) and incubated at 4°C for 1 hour. The plates were washed and 100$\mu$l/well of secondary antibody (anti-human Fc-488 antibody, Jackson ImmunoResearch, 109-546-098) diluted 1:1000 in PBS+2% BSA was then added and incubated for 30 mins at 4°C in the dark. The plates were washed and resuspended in 100 $\mu$l of PBS containing DAPI (Biotium, 40043) at 1 mg/ml. The plates were read using Canto II flow cytometer (BD Bioscience). Dead cells were excluded and the fluorescence in the FITC channel (488nm/530/30) was measured. The data was fitted using log (agonist) vs response in GraphPad Prism Software.

Example 2 - Conditional receptor agonism via mAb[2] crosslinking (mTNFRX-X orientation)

Activated T cells express the receptor TNFRX on their cell surface. Clustering of TNFRX is known to be essential for TNFRX signalling and further T cell activation. A T cell activation assay was used to assess clustering and signalling of the TNFRX receptor in the presence of the antibody molecules detailed below. T cell activation was determined by the release of IL-2.

2.1 Isolating and activating T cells

To isolate T cells, spleens were collected from 4-8 week old female Balb/C mice (Charles River). Mice were humanely euthanized and spleens were isolated by dissection. Spleenocytes were isolated by pushing the spleens

through a 70 $\mu$m cell strainer (Corning) using the inside of a 5 ml plastic syringe. The cell strainer was washed 10 times with 1ml Dulbecco's phosphate-buffered saline (DPBS) (Gibco) and the eluant collected in a 50ml tube. Red blood cells present in the eluant were lysed through the addition of 10 ml red blood cell lysis buffer (eBioscience) according to the manufacturer's instructions. T cells were isolated from the splenocytes present in the eluant using the pan T cell isolation kit II (Miltenyi Biotec Ltd) according to the manufacturer's instructions.

Mouse T-Activator CD3/CD28 Dynabeads (Life technologies) were resuspended by vortexing. Beads were washed twice with T cell medium (RPMI medium [Life Technologies] with 10% FBS [Life Technologies], 1x Penicillin Streptomycin [Life Technologies], Sodium Pyruvate [Gibco], 10mM Hepes [Gibco], 2mM L-Glutamine [Gibco] and 50$\mu$M 2-mercaptoethanol [Gibco]).

The required amount of T cells at a concentration of $1.0 \times 10^6$ cells/ml in T cell medium were stimulated with washed Mouse T-Activator CD3/CD28 Dynabeads (Life Technologies 11452D) at 2:1 cell to bead ratio in a T-25 flask (Sigma) and incubated overnight at 37°C + 5% $CO_2$ to activate the T cells.

2.2 Incubating T cells with CD-20 expressing Daudi cells

After overnight incubation, the activated T cells were washed from the Dynabeads and resuspended in T cell medium at a concentration of $2.0 \times 10^6$ cells/ml. 96-well flat-bottomed plates were coated with anti-mouse CD3 antibody through incubation with 2.5 $\mu$g/ml anti-mouse CD3 antibody (Biolegend) diluted in PBS for 2 hours at 37oC + 5% CO2 and then washed twice with PBS. Activated T cells were added to the plates at $1.0 \times 10^5$ cell/well. Daudi cells (ATCC, CCL-213), which express CD20 on their cell surface, were maintained in RPMI medium (Life Technologies) with 10% FBS (Life Technologies) and Sodium Pyruvate (Gibco). Daudi cells were washed once in T cell medium and added to the plates at $2.5 \times 10^4$ cells/well where needed.

2.3 Crosslinking mAb$^2$ molecules leads to effective induction of mTNFRX receptor clustering

The T cell activation assay was used to assess clustering and signalling of the TNFRX receptor in the presence of the antibody molecules detailed in Table 2 below.

A 2 $\mu$M dilution of each test antibody (see Table 2 for details) was prepared in DPBS (Gibco) and further diluted 1:10 in T cell medium (30 $\mu$l + 270 $\mu$l) to obtain a 200 nM dilution. The crosslinking agents (a-hFc, Jackson Immunoresearch; or FITCdex, Sigma) were added to the wells in a 1:1 molar ratio with the test antibodies where needed.

In a 96 well plate, 6 5-fold serial dilutions of the antibody or antibody/crosslinking antibody mixture were prepared (60 $\mu$l + 240 $\mu$l T cell medium). 100$\mu$l of the diluted antibody or antibody/crosslinking antibody mixture was added to the activated T cells on the plate.

T cells were incubated at 37°C, 5%$CO_2$ for 72 hours. Supernatants were collected and assayed with mouse IL-2 ELISA kit (eBioscience or R&D systems) following the manufacturer's instructions. Plates were read at 450 nm using the plate reader with the Gen5 Software, BioTek. Absorbance values of 630 nm were subtracted from those of 450 nm (Correction). The standard curve for calculation of cytokine concentration was based on a four parameter logistic curve fit (Gen5 Software, BioTek). The concentration of mouse IL-2 (mIL-2) was plotted vs the log concentration of antibody and the resulting curves were fitted using the log (agonist) vs response equation in GraphPad Prism. Table 3 shows the $EC_{50}$ values and maximum response of the IL-2 release observed in the T cell activation assay in the presence of the mAb$^2$ and mAbs tested. Figure 2 shows a representative plot of IL-2 release for the T cell activation assay.

Figure 2 shows that there is an increase in the activation of T cells when the TNFRX receptor is targeted and the anti-TNFRX antibodies are crosslinked. No T cell activation was observed with any of the antibodies at a single high concentration (100 nM) in the absence of crosslinking agents (Daudi cells, anti-Fc antibody or FITCdex) (data not shown). The anti-CD20 and anti-FITC antibodies do not induce T cell activation in the presence of crosslinking agents as expected. The anti-TNFRX antibody induces some T cell activation at high concentrations in the presence of Daudi cells (none/mTNFRX). This antibody induces higher levels of T cell activation and has lower $EC_{50}$ when crosslinked by an anti-Fc antibody (none/mTNFRX+ a-hFc) (see Table 3). The anti-TNFRX Fcab activates T cells when crosslinked by an anti-Fc antibody (mTNFRX/none + a-hFc) with a comparable $EC_{50}$ but lower maximum response than the TNFRX antibody (none/mTNFRX). The same anti-TNFRX Fcab when paired with an anti-FITC Fab in a mAb$^2$ activates T cells when crosslinked by FITCdex with a comparable $EC_{50}$ and maximum response to the TNFRX antibody (mTNFRX/FITC + FITCdex). The same anti-TNFRX Fcab when paired with an anti-CD20 Fab in a mAb$^2$ activates T cells in the presence of Daudi cells with a 100-fold lower $EC_{50}$ and comparable maximum response as the TNFRX antibody (mTNFRX/hCD20).

This data shows that the T cell activation observed only occurs upon crosslinking of an anti-TNFRX antibody, either using Daudi cells, an anti-human Fc antibody or by engagement of the Fab arms of a bispecific antibody. The lack of T cell activation in the absence of Daudi cells for the mTNFRX/hCD20 mAb$^2$ demonstrates that these molecules are conditional agonists that only induce T cell activation in the presence of the Fab target. This conditional agonism is present in both the mTNFRX/hCD20 and the mTNFRX/FITC mAb$^2$s demonstrating that both cell surface receptors and soluble factors can be used as Fab targets for crosslinking.

The crosslinking effect of Daudi cells on the TNFRX antibody (none/mTNFRX) occurs via Fc gamma receptor interaction and results in low levels of T cell activation and only at high antibody concentration. By contrast the mT-NFRX/hCD20 mAb[2] shows high levels of T cell activation at very low antibody concentration when the Fab arms are engaged by the CD20 receptor expressed at the surface of Daudi cells. This demonstrates that the mAb[2] format is superior for crosslinking an agonist receptor than an antibody (mAb) that relies on the presence of Fc gamma receptor expressing cells and TNFRX expressing cells within close proximity for antibody crosslinking and consequent receptor clustering.

2.4: Other cell surface receptors can be used to induce conditional receptor agonism via mAb[2] crosslinking

A T cell activation assay was used to assess clustering and signalling of the TNFRX receptor in the presence of the antibodies listed in the below Table 4.

T cells were isolated and activated as details in Example 2.1. After overnight incubation, the activated T cells were washed from the Dynabeads and resuspended in T cell medium at a concentration of $2.0 \times 10^6$ cells/ml. 96 well flat-bottomed plates were coated with anti-mouse CD3 antibody through incubation with 2.5 $\mu$g/ml anti-mouse CD3 antibody (Biolegend) diluted in PBS for 2 hours at 37°C + 5% $CO_2$ and then washed twice with PBS. Activated T cells were added to the plates at $1.0 \times 10^5$ cell/well. 4T1 cells (ATCC, CRL-2539), which express EpCAM and CD73 on their cell surface, were maintained in DMEM medium (Life Technologies) with 10% FBS (Life Technologies). 4T1 cells were washed once in T cell medium and added to the plates at $2.5 \times 10^4$ cells/well where needed.

A 2 $\mu$M dilution of each test antibody (see Table 4 for details) was prepared in DPBS (Gibco) and further diluted 1:10 in T cell medium (30 $\mu$l + 270 $\mu$l) to obtain a 200 nM dilution.

The crosslinking agents (a-hFc, Jackson Immunoresearch; or FITCdex, Sigma) were added to the wells in a 1:1 molar ratio with the test antibodies where needed.

In a 96-well plate, six five-fold serial dilutions of the antibody or antibody/crosslinking antibody mixture were prepared (60 $\mu$l + 240 $\mu$l T cell medium). 100$\mu$l of the diluted antibody or antibody/crosslinking antibody mixture was added to the activated T cells on the plate. T cells were incubated at 37°C, 5% $CO_2$ for 72 hours. Supernatants were collected and assayed with mouse IL-2 ELISA kit (eBioscience or R&D systems) following the manufacturer's instructions. Plates were read at 450 nm using the plate reader with the Gen5 Software, BioTek. Absorbance values of 630 nm were subtracted from those of 450 nm (Correction). The standard curve for calculation of cytokine concentration was based on a four parameter logistic curve fit (Gen5 Software, BioTek). The concentration of mouse IL-2 (mIL-2) was plotted vs the log concentration of antibody and the resulting curves were fitted using the log (agonist) vs response equation in GraphPad Prism. Table 5 shows the $EC_{50}$ values and maximum response of the IL-2 release observed in the T cell activation assay in the presence of the mAb[2] and mAbs tested. Figure 3 shows a representative plot of IL-2 release for the T cell activation assay.

Figure 3 shows that there is an increase in the activation of T cells when the TNFRX receptor is targeted and the anti-TNFRX antibodies are crosslinked. No T cell activation was observed with any of the antibodies at a single high concentration (100 nM) in the absence of crosslinking agents (4T1 cells, anti-Fc antibody or FITCdex) (data not shown). The anti-mouse EpCAM, anti-mouse CD73 and anti-FITC antibodies do not induce T cell activation in the presence of crosslinking agents as expected. The anti-mouse TNFRX antibody induces some T cell activation when crosslinked by an anti-Fc antibody (none/mTNFRX + a-hFc) (see Table 5). The anti-TNFRX Fcab when paired with an anti-FITC Fab in a mAb[2] activates T cells when crosslinked by FITCdex (mTNFRX/FITC + FITCdex) with a lower $EC_{50}$ and higher maximum response than the TNFRX antibody (none/mTNFRX). The same anti-TNFRX Fcab when paired with an anti-mouse EpCAM Fab or anti-mouse CD73 Fab in a mAb[2] activates T cells in the presence of 4T1 cells with a lower $EC_{50}$ and comparable maximum response as the TNFRX/FITC mAb[2] (mTNFRX/mEpCAM or mTNFRX/mCD73).

This data shows that the T cell activation observed only occurs upon crosslinking of an anti-TNFRX antibody, either using an anti-human Fc antibody or by engagement of the Fab arms of a bispecific antibody. The T cell activation observed with the mTNFRX/mEpCAM and mTNFRX/mCD73 mAb[2]s in the presence of 4T1 cells that express both mouse EpCAM and mouse CD73 demonstrates that more than one type of cell surface receptor can be used to pair with the anti TNFRX Fcabs, since CD73 is a GPI-linked cell surface receptor and EpCAM is a transmembrane glycoprotein.

Example 3 - mAb[2] crosslinking works in both orientations

A T cell activation assay was used to assess clustering and signalling of the TNFRX receptor in the presence of the antibodies listed in the below Table 6. T cell activation was determined by the release of IL-2.

3.1 Production of cell line over-expressing human EGFR

Lentiviral transduction methodology was used to generate CT26 cells (ATCC, CRL-2638) over-expressing human EGFR using the Lenti-X HTX Packaging System (Clontech, Cat. No 631249). Lenti-X expression vector (pLVX) (Clontech, Cat. No 631253), containing the human EGFR cDNA, was co-transfected with a Lenti-X HTX Packaging Mix into the Lenti-X 293T Cell Line (Clontech, Cat. No 632180) to generate virus. The CT26 cell line was transduced using the lentiviral vectors produced with the Lenti-X HTX Packaging System.

3.2 T cell activation assay

T cells were isolated and activated as set out in Example 2.1.

After overnight incubation, the activated T cells were washed from the Dynabeads and resuspended in T cell medium at a concentration of $2.0 \times 10^6$ cells/ml. 96 well flat-bottomed plates were coated with anti-mouse CD3 antibody through incubation with 2.5 $\mu$g/ml anti-mouse CD3 antibody (Biolegend) diluted in PBS for 2 hours at 37°C + 5% $CO_2$ and then washed twice with PBS. Activated T cells were added to the plates at $1.0 \times 10^5$ cell/well. CT26 cells (ATCC, CRL-2638), which have been transduced to express hEGFR on their cell surface, were maintained in DMEM medium (Life Technologies) with 10% FBS (Life Technologies). CT26.hEGFR cells were washed once in T cell medium and added to the plates at $2.5 \times 10^4$ cells/well where needed.

A 2 $\mu$M dilution of each test antibody (see Table 6 for details) was prepared in DPBS (Gibco) and further diluted 1:10 in T cell medium (30 $\mu$l + 270 $\mu$l) to obtain a 200 nM dilution.

The crosslinking agents (a-hFc, Jackson Immoresearch; or FITCdex, Sigma) were added to the wells in a 1:1 molar ratio with the test antibodies where needed.

In a 96-well plate, six five-fold serial dilutions of the antibody or antibody/crosslinking antibody mixture were prepared (60 $\mu$l + 240 $\mu$l T cell medium). 100$\mu$l of the diluted antibody or antibody/crosslinking antibody mixture was added to the activated T cells on the plate. T cells were incubated at 37°C, 5% $CO_2$ for 72 hours. Supernatants were collected and assayed with mouse IL-2 ELISA kit (eBioscience or R&D systems) following the manufacturer's instructions. Plates were read at 450 nm using the plate reader with the Gen5 Software, BioTek. Absorbance values of 630 nm were subtracted from those of 450 nm (Correction). The standard curve for calculation of cytokine concentration was based on a four parameter logistic curve fit (Gen5 Software, BioTek). The concentration of mouse IL-2 (mIL-2) was plotted vs the log concentration of antibody and the resulting curves were fitted using the log (agonist) vs response equation in GraphPad Prism.

3.3 Results of T cell activation assay

$EC_{50}$ values and maximum response of the IL-2 release observed in the T cell activation assay in the presence of the mAb[2] and mAbs tested are shown in Table 7. Figure 4 shows a representative plot of IL-2 release for the T cell activation assay.

Figure 4 shows that there is an increase in the activation of T cells when the TNFRX receptor is targeted and the anti-TNFRX antibodies are crosslinked. No T cell activation was observed with any of the antibodies at a single high concentration (100 nM) in the absence of crosslinking agents (CT26.hEGFR cells, anti-Fc antibody or FITCdex) (data not shown). The anti-EGFR (G1AA/Cx) mAb and anti-EGFR (FS1-67AA/4420) Fcab and anti-FITC antibodies (none/hEGFR) do not induce T cell activation in the presence of crosslinking agents as expected. The anti-TNFRX antibody induces T cell activation when crosslinked by an anti-Fc antibody (none/mTNFRX+ a-hFc) (see Table 7). The anti-TNFRX Fcab when paired with an anti-FITC Fab in a mAb[2] activates T cells when crosslinked by FITCdex with a lower $EC_{50}$ and higher maximum response to the TNFRX antibody (mTNFRX/FITC + FITCdex). The same anti-TNFRX Fcab when paired with an anti-EGFR Fab in a mAb[2] activates T cells in the presence of CT26.hEGFR cells with a lower $EC_{50}$ but lower maximum response to the TNFRX/FITC (mTNFRX/hEGFR). The TNFRX antibody when paired with an EGFR Fcab in a mAb[2] activates T cells in the presence of CT26.hEGFR cells with a lower $EC_{50}$ and comparable maximum response to the TNFRX/FITC (hEGFR/mTNFRX).

This data shows that both orientations of the mAb[2] can induce receptor clustering and T cell activation, so both the Fcab and Fab parts of the mAb[2] can target either TNFRX or a TAA type of receptor.

Example 4 - mAb[2] crosslinking with soluble factors

A T cell activation assay was used to determine whether soluble factors were able to induce clustering and signalling of the TNFRX receptor in the presence of the antibodies listed below in Table 8. 1mer is a soluble monomeric factor and 2mer is a soluble dimeric factor.

T cells were isolated and activated as described in Example 2.1.

After overnight incubation, the activated T cells were washed from the Dynabeads and resuspended in T cell medium at a concentration of $2.0 \times 10^6$ cells/ml. 96-well flat-bottomed plates were coated with anti-mouse CD3 antibody through incubation with 1 $\mu$g/ml anti-human CD3 antibody (Biotechne clone UCHT1) diluted in PBS for 2 hours at 37°C + 5% $CO_2$ and then washed twice with PBS. Activated T cells were added to the plates at $1.0 \times 10^5$ cell/well.

A 2 $\mu$M dilution of each test antibody (see Table 8 for details) was prepared in DPBS (Gibco) and further diluted 1:100 in T cell medium (3 $\mu$l + 297 $\mu$l) to obtain a 20 nM dilution.

The crosslinking agents (a-hFc, Jackson Immoresearch; or human 2mer Peprotech; or human 1mer) were added to the wells in a 1:1 molar ratio for anti-human Fc antibody and human 2mer or a 2:1 molar ratio for the 1mer with the test antibodies where needed. The diluted antibody or antibody/crosslinking antibody mixture (100$\mu$l/well) was added to the activated T cells on the plate.

T cells were incubated at 37°C, 5% $CO_2$ for 72 hours. Supernatants were collected and assayed with human IL-2 ELISA kit (eBioscience) following the manufacturer's instructions. Plates were read at 450 nm using the plate reader with the Gen5 Software, BioTek. Absorbance values of 630 nm were subtracted from those of 450 nm (Correction). The standard curve for calculation of cytokine concentration was based on a four parameter logistic curve fit (Gen5 Software, BioTek). Table 9 shows the human IL-2 levels produced in response to the presence of the mAb[2] and mAbs tested in the T cell activation assay. Figure 5 shows a representative plot of IL-2 release for the T cell activation assay.

Figure 5 shows that there is an increase in the activation of T cells when the TNFRX receptor is targeted and the anti-TNFRX antibodies are crosslinked. No T cell activation was observed with any of the antibodies at a single high concentration (10 nM) in the absence of crosslinking agents (anti-Fc antibody, 2mer or 1mer). The WT Fcab does not induce T cell activation in the presence of crosslinking agents as expected. The anti-TNFRX antibody induces T cell activation only when crosslinked with anti-human Fc antibody (none/hTNFRX + a-hFc). The same anti-human TNFRX Fab when paired with an Fcab targeting 1mer in a mAb[2] activates T cells when crosslinked by an anti-Fc antibody (1mer/hTNFRX + a-hFc) but not when crosslinked with either 2mer or 1mer (1mer/hTNFRX + 2mer and 1mer/hTNFRX + 1mer. The same anti-human TNFRX Fab when paired with an Fcab targeting 2mer in a mAb[2] activates T cells when crosslinked by an anti-Fc antibody (2mer/hTNFRX + a-hFc) and also when crosslinked with 2mer (2mer/hTNFRX + 2mer) but not soluble 1mer (2mer/hTNFRX + 1mer).

This data shows that soluble factors can be used to induce antibody crosslinking of a mAb[2] that targets TNFRX via the Fab arms and the soluble factor via the Fcab. This crosslinking results in T cell activation by clustering the TNFRX receptor on T cells. The lack of activity observed with the 1mer/TNFRX mAb[2] in the presence of 1mer suggests not all soluble factors are capable of inducing the antibody crosslinking effect required for T cell activation. The fact that 2mer is a dimer and 1mer is a monomer indicates the need for a multimeric soluble factor of at least n=2, where n indicates the number of subunits of the soluble factor.

Example 5 - Conditional receptor agonism of another TNF receptor (TNFRY)

A T cell activation assay was used to confirm that the mAb[2] format could be used to activate a different TNF receptor (TNFRY) expressed on T cells.

Cells expressing mouse TNFRY (DO11.10 cell line transfected with mouse TNFRY) were used in a T cell activation experiment in the presence or absence of hCD20 expressing Daudi cells (CD20+ cells). Details of the antibody molecules used in this experiment are shown in Table 10. Protein L was used as a cross-linking agent and was added along with mTNFRY monoclonal antibody (none/mTNFRY) in order to stimulate mTNFRY receptor activation.

T cell activation results in mIL-2 cytokine release, which was quantified by ELISA. Figure 6A and Figure 6B show the results for the T cell activation in the presence of various antibody molecules. The assay was performed for the mTNFRY antibody (none/mTNFRY) both in the absence (open squares, dashed line) and presence (closed squares, solid line) of hCD20 expressing cells, illustrated in Figure 6A and Figure 6B, respectively. The $EC_{50}$ results are shown in Table 11. In the absence of hCD20 expressing Daudi cells, mTNFRY/hCD20 mAb[2] results in minimal T cell activation (open circles, dashed line). The same mTNFRY/hCD20 mAb[2] activates T cells in the presence of hCD20 expressing Daudi cells (closed circles, solid line) resulting in a 30-fold lower $EC_{50}$ over the mTNFRY antibody in the presence of a cross-linking agent (none/mTNFRY). The addition of CD20+ cells resulted in similar levels of T-cell activation for the mTNFRY antibody (Figure 6B).

This experiment demonstrates that the mTNFRY/hCD20 mAb[2] shows high levels of T cell activation only when in the presence of hCD20 expressing cells, further demonstrating the suitability of the mAb[2] format as a conditional agonist. Furthermore, the mAb[2] shows high levels of T cell activation at low antibody concentration whereas high levels of mTNFRY antibody were required in order to drive T cell activation in the presence of an external cross-linking agent. This further demonstrates the superiority of the mAb[2] molecule at cross-linking an agonist receptor.

A hTNFRY/hCD20 mAb[2], i.e. a mAb[2] binding the corresponding human TNFRY antigen, as opposed to the murine TNFRY antigen bound by the mTNFRY/hCD20 mAb[2], was also tested in a T cell activation assay using the experimental set up described above. The assay was performed to test whether the hTNFRY/hCD20 mAb[2] could activate

T cells expressing the human version of TNFRY (DO11.10 cell line transfected with human TNFRY) in the presence or absence of hCD20 expressing Daudi cells (CD20⁺ cells). The results obtained were comparable to the results obtained with the mTNFRY/hCD20 mAb² described above.

<u>Example 6 - Other cell surface receptors can be used to induce conditional receptor agonism via mAb² crosslinking *in vitro* and *in vivo*</u>

*6.1. Other cell surface receptors can be used to induce conditional receptor agonism via mAb² crosslinking in vitro*

A T cell activation assay was used to assess clustering and signalling of the TNFRX receptor in the presence of the test antibodies listed in Table 12 below.

T cells were isolated and activated as detailed in Example 2.1. After overnight incubation, the activated T cells were washed from the Dynabeads and resuspended in T cell medium at a concentration of $2.0 \times 10^6$ cells/ml. 96-well flat-bottomed plates were coated with anti-mouse CD3 antibody through incubation with 2.5 $\mu$g/ml anti-mouse CD3 antibody (Biolegend) diluted in PBS for 2 hours at 37°C + 5% $CO_2$ and then washed twice with PBS. Activated T cells were added to the plates at $1.0 \times 10^5$ cell/well. HPAC cells (ATCC, CRL2119™), which express human EphA2 on their cell surface, were maintained in DMEM medium (Life Technologies) with 10% FBS (Life Technologies). HPAC cells were washed once in T cell medium and added to the plates at $2.5 \times 10^4$ cells/well where needed.

A 2 $\mu$M dilution of each test antibody (see Table 12 for details) was prepared in DPBS (Gibco) and further diluted 1:10 in T cell medium (30 $\mu$l + 270 $\mu$l) to obtain a 200 nM dilution.

The crosslinking agents (a-hFc, Jackson Immunoresearch; or FITCdex, Sigma) were added to the wells in a 1:1 molar ratio with the test antibodies where needed.

In a 96-well plate, six five-fold serial dilutions of the antibody or antibody/crosslinking antibody mixture were prepared (60 $\mu$l + 240 $\mu$l T cell medium). 100 $\mu$l of the diluted antibody or antibody/crosslinking antibody mixture was added to the activated T cells on the plate. T cells were incubated at 37°C, 5% $CO_2$ for 72 hours. Supernatants were collected and assayed with mouse IL-2 ELISA kit (eBioscience or R&D Systems) following the manufacturer's instructions. Plates were read at 450 nm using the plate reader with the Gen5 Software, BioTek. Absorbance values of 630 nm were subtracted from those of 450 nm (Correction). The standard curve for calculation of cytokine concentration was based on a four parameter logistic curve fit (Gen5 Software, BioTek). The concentration of mouse IL-2 (mIL-2) was plotted versus the log concentration of antibody and the resulting curves were fitted using the log (agonist) versus response equation in GraphPad Prism. Table 13 shows the $EC_{50}$ values and maximum response of the IL-2 release observed in the T cell activation assay in the presence of the mAb² and mAbs tested. Figures 7 A and B show a representative plot of IL-2 release for the T cell activation assay.

Figure 7A shows that there is an increase in the activation of T cells in the presence of EphA2 expressing cells (HPAC) when a bispecific antibody targeting the TNFRX receptor and the EphA2 receptor is present but not when other antibodies targeting the TNRFX receptor are present but not crosslinked. This indicates that the bispecific antibody is crosslinked by binding to the two targets, TNFRX and EphA2. Figure 7B shows that any antibodies targeting the TNFRX receptor activate T cells in the presence of non-physiological crosslinking agents (anti-Fc antibody or FITCdex). The anti-EphA2 and anti-FITC antibodies do not induce T cell activation in the presence of crosslinking agents, as expected. The anti-mouse TNFRX antibody induces some T cell activation when crosslinked by an anti-Fc antibody (none/mTNFRX + a-hFc) (see Table 13). The anti-TNFRX Fcab when paired with an anti-FITC Fab in a mAb² activates T cells when crosslinked by FITCdex (mTNFRX/FITC + FITCdex) with a lower $EC_{50}$ and higher maximum response than the TNFRX antibody (none/mTNFRX). The same anti-TNFRX Fcab when paired with an anti-EphA2 Fab in a mAb² (mTNFRX/EphA2) activates T cells in the presence of HPAC cells with a lower $EC_{50}$ and comparable maximum response as compared to the mTNFRX/FITC mAb².

This data shows that the T cell activation observed only occurs upon crosslinking of an anti-TNFRX antibody, either using an anti-human Fc antibody or by engagement of the Fab arms of a bispecific antibody. The T cell activation observed with the mTNFRX/EphA2 mAb² in the presence of HPAC cells expressing EphA2 demonstrates that this receptor can also mediate crosslinking of the mTNFRX receptor when targeted by a mAb² bispecific antibody with specificities against EphA2 and mTNRFX.

A T cell activation experiment to test the ability of the mTNFRX Fcab paired with an anti-human PSMA Fab in mAb² format (mTNFRX/hPSMA) to activate T cells expressing mTNFRX in the presence or absence of cells expressing the cell surface tumour antigen hPSMA was also performed and gave comparable results to those obtained from the T cell activation assays for the mTNFRX/hEpCAM mAb² described above, as well as the mTNFRX/hCD20, mTNFRX/mCD73, mTNFRX/hEGFR, and mTNFRX/VEGF mAb² pairings described elsewhere herein.

Similar T cell activation assays were also performed to test whether mAb² in which the Fcab bound to human TNFRX, rather than murine TNFRX, could activate T cells in a T cell activation assay. The molecules tested were hTNFRX/mEpCAM, hTNFRX/hEGFR, hTNFRX/EphA2 and hTNFRX/hCEACAM5 mAb². The T cells used in the T cell activation assay expressed the human version of TNFRX, and were tested in the presence or absence of cells expressing the relevant cell surface tumour antigens bound by the Fab portion of the mAb[2], i.e. mEpCAM, hEGFR, EphA2 or hCEACAM5, respectively. The results were comparable to those obtained in T cell activation assays with mAb[2] molecules incorporating the mTNFRX Fcab, and Fab portions binding to hCD20, hEpCAM, mCD73, hEGFR, EphA2, VEGF or hPSMA, as described herein (i.e. mTNFRX/hCD20, mTNFRX/hEpCAM, mTNFRX/mCD73, mTNFRX/hEGFR, mTNFRX/EphA2, mTNFRX/VEGF and mTNFRX/hPSMA).

*6.2 mAb[2] capable of conditional receptor agonism in vitro supress tumour growth in vivo*

The CT26 syngeneic tumour model was used in this experiment as CT26 cells express murine EphA2 and tumour infiltrating lymphocytes (TILs) isolated from CT26 tumours express mTNFRX. The mTNFRX/EphA2 mAb[2] was tested for *in vivo* activity in a CT26 syngeneic mouse tumour growth model. The ability of the mAb[2] to inhibit tumour growth was compared to that of the mTNRFX/none mAb[2], the combination of mTNFRx/none mAb[2] and none/EphA2 mAb, and the none/FITC mAb controls. BALB/c female mice (Charles River) aged 8-10 weeks and weighing 20-25 g each were rested for one week prior to the study start. All animals were micro-chipped and given a unique identifier. Each cohort had 12 mice. The CT26 colon carcinoma cell line (ATCC, CRL-2638) was initially expanded, stored, and then pre-screened by IDEXX Bioresearch for pathogens using the IMPACT I protocol and shown to be pathogen free. CT26 cells (approximately $3\text{-}5 \times 10^6$) were thawed from -150°C storage and added to 20 ml DMEM (Gibco, 61965-026) with 10% FCS (Gibco, 10270-106) in a T175 tissue culture flask. Mice were anaesthetised using isoflurane (Abbott Laboratories) and each animal received $1 \times 10^6$ cells injected subcutaneously in the left flank. On day 10 following tumour cell inoculation, mice were monitored for health and tumour growth and were sorted and randomised into study cohorts. Any mice which did not have tumours at this point were removed from the study.

All of the mAb[2] molecules and the control antibodies were analysed within 24 hours of injection by SEC-HPLC profiling and checked for impurities. Antibodies were prepared at final concentration of 1 mg/kg in PBS. The mAb[2] molecules and the control antibodies were administered to the mice by intraperitoneal (IP) injection on days 10, 12 and 14 following tumour inoculation. Animals were health screened under anaesthesia three times a week in a blinded fashion, during which time accurate measurements of tumours were taken. Tumour volume measurements were taken with callipers to determine the longest axis and the shortest axis of the tumour. The following formula was used to calculate the tumour volume:

$$L \times (S^2) / 2$$

(Where L = longest axis; S= shortest axis)

The trial was halted at day 20 when the tumour burden was considered close to restrictions and all mice were humanely sacrificed. The results are shown in Figure 8. Statistical analysis of the end tumour volumes was performed using a two tailed Student's t-test within the GraphPad Prism software package.

There was a demonstrated statistically significant difference between mTNRFX/EphA2 mAb[2] and none/FITC control (normal growth) in suppressing tumour growth. Such a statistically significant difference was not observed with either the combination of mTNFRX/none and none/EphA2 or mTNFRX/none versus none/FITC control group.

The CT26 tumour model is an aggressive, fast growing tumour model, one that is inherently prone to mice developing intestinal metastasis, and as a result has a very limited therapeutic window. Surprisingly, the combination of antibodies targeting mTNFRX and EphA2 did not significantly suppress tumour growth compared to the IgG control cohort. However, the mTNFRX/EphA2 treated cohort did reveal a significant suppression of growth compared to IgG control. This trial shows that, similar to the observed *in vitro* results, the crosslinking of mTNFRX by a mAb[2] bispecific antibody targeting the EphA2 expressed in the tumour cells and the mTNRFX expressed in TILs results in T cell activation and subsequent tumour growth control above what is observed with controls.

Example 7 - *in vivo* anti-tumour activity of a mTNFRY/EGFR mAb[2]

7.1 Production of a cell line over-expressing human EGFR by retroviral transduction

Retroviral transduction methodology was used to generate CT26 cells (ATCC, CRL-2638) over-expressing human EGFR using the MSCV Retroviral Expression System (Clontech, Cat. No 634401). MSCV expression vector (pMSCV-hyg) (Clontech, Cat. No 631461), containing the human EGFR cDNA, was transfected into the RetroPack PT67 cell line (Clontech, Cat. No 631510) to generate virus. The CT26 cell line was transduced using the retroviral vectors produced with the MSCV Retroviral Expression System.

7.2 *in vivo* anti-tumour activity of a mAb[2]

The CT26.hEGFR syngeneic tumour model was produced by transducing a human EGFR expressing retrovirus into CT26 cells as described in 7.1 above. These cells were tested for their *in vivo* growth properties and shown to grow comparably to wild-type CT26 cells. These cells were then used to test the *in vivo* anti-tumour activity of a mTNFRY/hE-GFR mAb[2]. The ability of the mAb[2] to inhibit tumour growth was compared to that of the following controls: a combination of a mTNFRY/none mAb[2] and a none/hEGFR mAb; a none/FITC mAb; and the none/hEGFR mAb alone.

BALB/c female mice (Charles River) aged 8-10 weeks and weighing 20-25 g each were rested for one week prior to the study start. All animals were micro-chipped and given a unique identifier. Each cohort had 12 mice. CT26.hE-GFR cells (approximately $3\text{-}5 \times 10^6$) were thawed from -150°C storage and added to 20 ml DMEM (Gibco, 61965-026) with 10% FCS (Gibco, 10270-106) and 100 ug/ml hygromycin (Gibco, 10687010) in a T175 tissue culture flask. Mice were anaesthetised using isoflurane (Abbott Laboratories) and each animal received $1 \times 10^6$ cells injected subcutaneously in the left flank. On day 7 following tumour cell inoculation, mice were monitored for health and tumour growth and were sorted and randomised into study cohorts. Any mice which did not have tumours at this point were removed from the study.

All of the mAb[2] molecules and the control antibodies were analysed within 24 hours of injection by SEC-HPLC profiling and checked for impurities. Antibodies were prepared at final concentration of 1 mg/kg in PBS. The mAb[2] molecules and the control antibodies were administered to the mice by intraperitoneal (IP) injection on days 7, 9 and 11 following tumour inoculation. Animals were health screened under anaesthesia three times a week in a blinded fashion, during which time accurate measurements of tumours were taken. Tumour volume measurements were taken with callipers to determine the longest axis and the shortest axis of the tumour. The following formula was used to calculate the tumour volume:

$$L \times (S^2) / 2$$

(Where L = longest axis; S= shortest axis)

The trial was halted at day 20 when the tumour burden was considered close to restrictions and all mice were humanely sacrificed. The results are shown in Figure 9. Statistical analysis of the end tumour volumes was performed using a two tailed Student's t-test within the GraphPad Prism software package.

There was a demonstrated statistically significant difference between mTNRFY/hEGFR mAb[2] and the none/hE-GFR control but not with the other groups. It is unclear why the none/EGFR control group shows a slightly faster tumour growth than the none/FITC control. However, it is clear that the mTNFRY/hEGFR mAb[2] shows suppression of tumour growth in the CT26.hEGFR tumour model. The combination of mTNFRY/none and none/hEGFR control versus the none/hEGFR control did not show a statistically significant difference.

The CT26.hEGFR tumour model grows *in vivo* at the same rate as the CT26 tumour model, which is an aggressive, fast growing tumour model, one that is inherently prone to mice developing intestinal metastasis, and as a result has a very limited therapeutic window. Surprisingly, the combination of antibodies targeting mTNFRY and hEGFR did not significantly suppress tumour growth compared to the IgG control cohort. However, the mTNFRY/hEGFR treated cohort did reveal a significant suppression of growth compared to the none/hEGFR control.

Example 8 - Soluble factors can be used to induce conditional receptor agonism via mAb[2] crosslinking *in vivo, as well as in vitro*

8.1 Soluble factors can be used to induce conditional receptor agonism via mAb[2] crosslinking *in vitro*

To determine whether the set of antibodies tested in this Example were able to induce clustering and signalling of the TNFRX receptor *in vitro,* they were tested as described for the antibodies tested in Example 4 above.

Specifically, a T cell activation assay was used to assess clustering and signalling of the TNFRX receptor in the presence of the antibodies listed in Table 14 below.

T cells were isolated and activated as detailed in Example 2.1.

After overnight incubation, the activated T cells were washed from the Dynabeads and resuspended in T cell medium at a concentration of $2.0 \times 10^6$ cells/ml. 96-well flat-bottomed plates were coated with anti-mouse CD3 antibody through incubation with 2.5 $\mu$g/ml anti-mouse CD3 antibody (Biolegend) diluted in PBS for 2 hours at 37°C + 5% $CO_2$ and then washed twice with PBS. Activated T cells were added to the plates at $1.0 \times 10^5$ cells/well.

A 2 $\mu$M dilution of each test antibody (see Table 15 for details) was prepared in DPBS (Gibco) and further diluted 1:10 in T cell medium (30 $\mu$l + 270 $\mu$l) to obtain a 200 nM dilution.

The crosslinking agents (a-hFc, Jackson Immunoresearch; FITCdex, Sigma; or murine VEGF 165, Peprotech) were added to the wells in a 1:1 molar ratio with the test antibodies where needed.

In a 96-well plate, six five-fold serial dilutions of the antibody or antibody/crosslinking antibody mixture were prepared (60 μl + 240 μl T cell medium). 100 μl of the diluted antibody or antibody/crosslinking antibody mixture was added to the activated T cells on the plate. T cells were incubated at 37°C, 5% $CO_2$ for 72 hours. Supernatants were collected and assayed with mouse IL-2 ELISA kit (eBioscience or R&D Systems) following the manufacturer's instructions. Plates were read at 450 nm using the plate reader with the Gen5 Software, BioTek. Absorbance values of 630 nm were subtracted from those of 450 nm (Correction). The standard curve for calculation of cytokine concentration was based on a four parameter logistic curve fit (Gen5 Software, BioTek). Table 15 shows the human IL-2 levels produced in response to the presence of the mAb[2] and mAbs tested in the T cell activation assay. Figure 10 shows a representative plot of IL-2 release for the T cell activation assay. In Figure 11, the concentration of mouse IL-2 (mIL-2) was plotted versus the log concentration of antibody and the resulting curves were fitted using the log (agonist) versus response equation in GraphPad Prism. Table 16 shows the $EC_{50}$ values and maximum response of the IL-2 release observed in the T cell activation assay in the presence of the mAb[2] and mAbs tested.

Figure 10 shows the activity of the different antibodies in the presence of the different crosslinking agents at a single concentration (10nM). This figure shows that there is an increase in the activation of T cells only when mTNFRX targeting antibodies are crosslinked.

The control antibodies (none/mTNFRX and mTNFRX/FITC) can be crosslinked by non-physiological crosslinkers (anti-hFc, FITCdex) but not by VEGF, whereas the mTNFRX/VEGF mAb[2] is crosslinked by VEGF which represents a physiological soluble factor that can be overexpressed at a tumour site. The higher background observed with the mTNFRX/VEGF is likely to be the result of residual VEGF produced by activated T cells (J. Immunol. 2004 Apr 1;172(7):4618-23.). The anti-VEGF and anti-FITC antibodies do not induce T cell activation in the presence of crosslinking agents as expected. When the crosslinked activity of the mTNFRX/VEGF mAb[2] is compared to the control mTNFRX/FITC mAb[2] by titrating the antibody concentration, as shown in Figure 11 both the $EC_{50}$ and the maximum response of the two mAb[2] bispecific antibodies are comparable (see Table 16). The mTNFRX/VEGF mAb[2] has some background activity at higher concentrations (10nM) but this is decreased at lower concentrations (e.g. 1nM), whereas the activity of the mAb[2] crosslinked with a 1:1 VEGF ratio is maintained at these lower concentrations.

This data shows that the T cell activation observed only occurs upon crosslinking of an anti-TNFRX antibody. The crosslinking can be the result of a secondary antibody (a-hFc) binding to more than one mTNFRX targeting antibody, for example none/mTNFRX + a-hFc, and clustering the TNFRX this way. It can also be the result of engaging both antigen-binding ends of a mAb[2] bispecific antibody, one end targeting TNFRX and the other targeting a soluble factor that can be overexpressed at a tumour site, such as VEGF.

8.2 mAb[2] capable of conditional receptor agonism *in vitro* supress tumour growth *in vivo*

The mTNFRX/VEGF mAb[2] was tested for *in vivo* activity in a CT26 syngeneic mouse tumour growth model.

The CT26 syngeneic tumour model was used in this experiment, as CT26 tumours have been described to have an increased concentration of VEGF (Voron T, Colussi O, Marcheteau E, et al. VEGF-A modulates expression of inhibitory checkpoints on CD8+ T cells in tumors. The Journal of Experimental Medicine. 2015;212(2):139-148) and tumour infiltrating lymphocytes (TILs) isolated from CT26 tumours express mTNFRX.

The ability of the mTNFRX/VEGF mAb[2] to inhibit tumour growth was compared to that of the following controls: a combination of a mTNFRX/none mAb[2] and a none/VEGF mAb; a none/FITC mAb; and the none/VEGF mAb alone.

BALB/c female mice (Charles River) aged 8-10 weeks and weighing 20-25 g each were rested for one week prior to the study start. All animals were micro-chipped and given a unique identifier. Each cohort had 15 mice. The CT26 colon carcinoma cell line (ATCC, CRL-2638) was initially expanded, stored, and then pre-screened by IDEXX Bioresearch for pathogens using the IMPACT I protocol and shown to be pathogen free. CT26 cells (approximately 3-5 × 10[6]) were thawed from -150°C storage and added to 20 ml DMEM (Gibco, 61965-026) with 10% FCS (Gibco, 10270-106) in a T175 tissue culture flask. Mice were anaesthetised using isoflurane (Abbott Laboratories) and each animal received 1 × 10[6] cells injected subcutaneously in the left flank. On day 10 following tumour cell inoculation, mice were monitored for health and tumour growth and were sorted and randomised into study cohorts. Any mice which did not have tumours at this point were removed from the study.

All of the mAb[2] molecules and the control antibodies were analysed within 24 hours of injection by SEC-HPLC profiling and checked for impurities. Antibodies were prepared at final concentration of 1 mg/kg in PBS. The mAb[2] molecules and the control antibodies were administered to the mice by intraperitoneal (IP) injection on days 10, 12 and 14 following tumour inoculation. Animals were health screened under anaesthesia three times a week in a blinded fashion, during which time accurate measurements of tumours were taken. Tumour volume measurements were taken with callipers to determine the longest axis and the shortest axis of the tumour. The following formula was used to calculate the tumour volume:

$$L \times (S^2) / 2$$

(Where L = longest axis; S= shortest axis)

The trial was halted at day 24 when the tumour burden was considered close to restrictions and all mice were humanely sacrificed. The results are shown in Figure 12. Statistical analysis of the end tumour volumes was performed using a two tailed Student's t-test within the GraphPad Prism software package.

There was a demonstrated statistically significant difference between mTNRFX/VEGF mAb[2] and none/FITC control (normal growth) in suppressing tumour growth. Such a statistically significant difference was not observed with either the combination of mTNFRX/none and none/VEGF control or the none/VEGF control versus the none/FITC control.

The CT26 tumour model is an aggressive, fast growing tumour model, one that is inherently prone to mice developing intestinal metastasis, and as a result has a very limited therapeutic window. Surprisingly, the combination of antibodies targeting mTNFRX and VEGF did not significantly suppress tumour growth compared to the IgG control cohort. However, the mTNFRX/VEGF treated cohort did reveal a significant suppression of growth compared to IgG control. This trial shows that, similar to the observed *in vitro* results, the crosslinking of mTNFRX by a mAb[2] bispecific antibody targeting the VEGF overexpressed by tumour cells or within or at the tumour microenvironment and the mTNRFX expressed in TILs results in T cell activation and subsequent tumour growth control above what is observed with controls.

**Table 1:** Details of antibody molecules used to demonstrate conditional agonism

| Label (Fcab/Fab target) | Molecule type | Fab binding to | Fab Reference | Fab clone | Fcab binding to | LALA mutation |
|---|---|---|---|---|---|---|
| none/none | Fcab | none | N/A | N/A | none | Yes |
| mTNFRX/none | Fcab | none | N/A | N/A | mTNFRX | Yes |
| none/FITC | mAb | FITC | Bedzyk, W. D., et al. J. Biol. Chem 264: 1565-1569 (1989) | 4420 | none | Yes |
| mTNFRX/FITC | mAb[2] | | | | mTNFRX | Yes |
| hEGFR/FITC | mAb[2] | | | | hEGFR | Yes |
| none/hCD20 | mAb | hCD20 | US 8529902 B2 | 2F2 | none | Yes |
| mTNFRX/hCD20 | mAb[2] | | | | mTNFRX | Yes |
| none/mEpCAM | mAb | mEPCAM | Farr A, et al. J. Histochem. Cytochem. 39:645. (1991) | G8.8 | none | Yes |
| mTNFRX /mEpCAM | mAb[2] | | | | mTNFRX | Yes |
| none/hEpCAM | mAb | hEPCAM | US 8637017 B2 | MOC31 | none | Yes |
| mTNFRX /hEpCAM | mAb[2] | | | | mTNFRX | Yes |
| none/mCD73 | mAb | mCD73 | Yamashita, et al. Eur J Immunol. 28(10): 2981-90. (1998) | TY11.8 | none | Yes |
| mTNFRX /mCD73 | mAb[2] | | | | mTNFRX | Yes |
| none/hEGFR | mAb | hEGFR | US 6217866 B1 | cetuximab | none | Yes |
| mTNFRX/hEGFR | mAb[2] | | | | mTNFRX | Yes |
| none/mTNFRX | mAb | mTNFRX | | | none | Yes |
| hEGFR/mTNFRX | mAb[2] | | | | hEGFR | Yes |
| none/hTNFRX | mAb | hTNFRX | | | none | Yes |
| 2mer/hTNFRX | mAb[2] | | | | 2mer | Yes |
| 1mer/hTNFRX | mAb[2] | | | | 1mer | Yes |
| mTNFRY/hCD20 | mAb[2] | hCD20 | US 8529902 B2 | 2F2 | mTNFRY | Yes |
| none/mTNFRY | mAb | mTNFRY | | | none | Yes |
| hTNFRY/hCD20 | mAb[2] | hCD20 | US 8529902 B2 | 2F2 | hTNFRY | Yes |

(continued)

| Label (Fcab/Fab target) | Mole cule type | Fab binding to | Fab Reference | Fab clone | Fcab binding to | LALA mutation |
|---|---|---|---|---|---|---|
| none/EphA2 (human and mouse EphA2 cross-reactive) | mAb | EphA2 | WO 2004/014292 A2 | EA2 | none | Yes |
| mTNFRX/EphA2 (human and mouse EphA2 cross-reactive) | mAb$^2$ | | | | mTNFRX | Yes |
| none/hEGFR | mAb | hEGFR | US 6217866 B1 | cetuximab | none | No |
| mTNFRY/none (HeID1.3) | mAb$^2$ | none | | | mTNFRY | Yes |
| mTNFRY/hEGFR | mAb$^2$ | hEGFR | US 6217866 B1 | cetuximab | mTNFRY | Yes |
| none/VEGF (human and mouse VEGF cross-reactive) | mAb | VEGF | US 2009/0175791 A1 | r84 | none | Yes |
| mTNFRX/VEGF (human and mouse VEGF cross-reactive) | mAb$^2$ | VEGF | US 2009/0175791 A1 | r84 | mTNFRX | Yes |
| mTNFRX/hPSMA | mAb$^2$ | hPSMA | US8461308B2 | 2A10 | mTNFRX | Yes |
| hTNFRX/hCEACA M5 | mAb$^2$ | hCEACAM5 | US8771690 B2 | hMN15 | hTNFRX | Yes |
| hTNFRX/hEGFR | mAb$^2$ | hEGFR | US 6217866 B1 | cetuximab | hTNFRX | Yes |
| hTNFRX/EphA2 (human and mouse EphA2 cross-reactive) | mAb$^2$ | EphA2 | WO 2004/014292 A2 | EA2 | hTNFRX | Yes |
| hTNFRX/hEpCAM | mAb$^2$ | hEpCAM | US 8637017 B2 | MOC31 | hTNFRX | Yes |

**Table 2:** Details of antibody molecules tested

| Test antibody molecule (Fcab/Fab target) | Molecule type | Fab target | Fab clone | Fcab target |
|---|---|---|---|---|
| none/mTNFRX | mAb | mTNFRX | | none |
| none/hCD20 | mAb | hCD20 | 2F2 | none |
| mTNFRX/hCD20 | mAb$^2$ | hCD20 | 2F2 | mTNFRX |
| mTNFRX/FITC | mAb$^2$ | FITC | 4420 | mTNFRX |
| mTNFRX/none | Fcab | none | n/a | mTNFRX |
| none/FITC | mAb | FITC | 4420 | none |

**Table 3:** EC$_{50}$ and maximum response values of mAb and mAb$^2$ molecules in the T cell activation assay.

| Antibody molecule + crosslinking agent (in the presence of Daudi cells) | EC$_{50}$ (nM) | EC$_{50}$ 95% Conf. Int. | Max response (mIL-2 pg/ml) | Max response 95% Conf. Int. |
|---|---|---|---|---|
| none/FITC + FITCdex | 0.002124 | 6.914e-006 to 0.6524 | 529.5 | 373.9 to 685.1 |

(continued)

| Antibody molecule + crosslinking agent (in the presence of Daudi cells) | EC$_{50}$(nM) | EC$_{50}$ 95% Conf. Int. | Max response (mIL-2 pg/ml) | Max response 95% Conf. Int. |
|---|---|---|---|---|
| none/hCD20 + a-hFc | 3.029 | 1.401e-005 to 654848 | 561.6 | 199.9 to 923.3 |
| none/mTNFRX | 126 | 4.474 to 3549 | 10520 | -7314 to 28354 |
| none/mTNFRX + a-hFc | 3.008 | 2.003 to 4.519 | 15586 | 14390 to 16782 |
| mTNFRX/none + a-hFc | 0.9199 | 0.3890 to 2.175 | 10386 | 8843 to 11929 |
| mTNFRX/FITC + FITCdex | 0.4347 | 0.2822 to 0.6697 | 15873 | 14806 to 16941 |
| mTNFRX/hCD20 | 0.02538 | 0.01186 to 0.05432 | 15126 | 13845 to 16407 |

**Table 4:** Details of antibody molecules tested

| Test antibody molecule (Fcab/Fab target) | Molecule type | Fab target | Fab clone | Fcab target |
|---|---|---|---|---|
| none/FITC | mAb | FITC | 4420 | none |
| mTNFRX/FITC | mAb$^2$ | FITC | 4420 | mTNFRX |
| none/mEpCAM | mAb | mEpCAM | G8.8 | none |
| mTNFRX/mEpCAM | mAb$^2$ | mEpCAM | G8.8 | mTNFRX |
| none/mCD73 | mAb | mCD73 | TY11.8 | none |
| mTNFRX/mCD73 | mAb$^2$ | mCD73 | TY11.8 | mTNFRX |
| none/mTNFRX | mAb | mTNFRX | | none |

**Table 5**: EC$_{50}$ and maximum response values of mAb and mAb$^2$ molecules in the T cell activation assay.

| Antibody molecule + crosslinking agent (in the presence of 4T1 cells) | EC$_{50}$ (nM) | EC$_{50}$ 95% Conf. Int. | Max response (mIL-2 pg/ml) | Max response 95% Conf. Int. |
|---|---|---|---|---|
| none/FITC + FITCdex | 4.354 | 0.0 to 9.348e+024 | -32 | -137.0 to 72.98 |
| mTNFRX/FITC + FITCdex | 0.7818 | 0.3069 to 1.991 | 5397 | 4551 to 6243 |
| none/mEpCAM | 0.3026 | 3.724e-006 to 24590 | 44.24 | -56.07 to 144.5 |
| mTNFRX/mEpCAM | 0.01701 | 0.002492 to 0.1161 | 6912 | 6200 to 7623 |
| none/mCD73 | 0.1718 | 0.0007464 to 39.54 | 54.99 | -8.714 to 118.7 |
| mTNFRX/mCD73 | 0.005914 | 0.0001521 to 0.2300 | 5299 | 4653 to 5945 |
| none/TNFRX + a-hFc | 1.562 | 0.1921 to 12.71 | 1439 | 855.1 to 2024 |

**Table 6:** Details of antibodies and mAb[2] tested

| Test antibody molecule (Fcab/Fab target) | Molecule type | Fab target | Fab clone | Fcab target |
|---|---|---|---|---|
| none/FITC | mAb | FITC | 4420 | none |
| mTNFRX/FITC | mAb[2] | FITC | 4420 | mTNFRX |
| none/mTNFRX | mAb | mTNFRX | | none |
| hEGFR/FITC | mAb[2] | FITC | 4420 | hEGFR |
| hEGFR/mTNFRX | mAb[2] | mTNFRX | mTNFRX | hEGFR |
| none/hEGFR | mAb | hEGFR | cetuximab | none |
| mTNFRX/hEGFR | mAb[2] | hEGFR | cetuximab | mTNFRX |

**Table 7:** $EC_{50}$ and maximum response values of mAb and mAb[2] molecules in the T cell activation assay.

| Fcab/Fab targets (in the presence of CT26.hEGFR) | $EC_{50}$ (nM) | $EC_{50}$ 95% Conf. Int. | Max response (mIL-2 pg/ml) | Max response 95% Conf. Int. |
|---|---|---|---|---|
| none/FITC + FITCdex | 10.17 | 0.001009 to 102513 | 5.152 | -23.09 to 33.39 |
| mTNFRX/FITC + FITCdex | 0.7472 | 0.05491 to 10.17 | 2813 | 1638 to 3988 |
| none/mTNFRX + a-hFc | 2.695 | 0.2655 to 27.35 | 842.1 | 463.6 to 1221 |
| hEGFR/FITC + FITCdex | 12040000 | | -1925000 | |
| hEGFR/mTNFRX | 0.1267 | 0.02432 to 0.6598 | 2533 | 2112 to 2954 |
| none/hEGFR + a-hFc | 1.657E-07 | | -8.769 | |
| mTNFRX/hEGFR | ~ 1.854e-005 | (Very wide) | 1081 | 734.1 to 1429 |

**Table 8:** Details of antibody molecules tested

| Test antibody molecule (Fcab/Fab target) | Molecule type | Fab target | Fcab target |
|---|---|---|---|
| none/none | Fcab | none | none |
| none/hTNFRX | mAb | hTNFRX | none |
| 2mer/hTNFRX | mAb[2] | hTNFRX | 2mer |
| 1mer/hTNFRX | mAb[2] | hTNFRX | 1mer |

**Table 9:** Human IL-2 produced in response to mAb and mAb[2] molecules in the T cell activation assay.

| hIL-2 (pg/ml) | Average | | | | Standard deviation | | | |
|---|---|---|---|---|---|---|---|---|
| | none | a-hFc | 2mer | 1mer | none | a-hFc | 2mer | 1mer |
| WT Fcab | 61.25 | 17.5 | -4.37 | -7.5 | 70.71 | 8.84 | 39.77 | 35.36 |
| none/hTNFRX | 61.25 | 1348.75 | 3298.75 | 1983.13 | 35.36 | 1856.16 | 901.56 | 2762.14 |
| 1mer/hTNFRX | 98.75 | 1461.25 | 2598.75 | 1264.38 | 70.71 | 1856.16 | 247.49 | 1639.6 |
| 2mer/hTNFRX | 70.63 | 1305 | 2348.75 | 2483.13 | 22.1 | 1767.77 | 291.68 | 481.72 |

**Table 10:** Details of antibody molecules tested

| Test antibody molecule (Fcab/Fab target) | Molecule type | Fab target | Fab clone | Fcab target |
|---|---|---|---|---|
| none/mTNFRY | mAb | mTNFRY | | none |
| none/hCD20 | mAb | hCD20 | 2F2 | none |
| mTNFRY/hCD20 | mAb$^2$ | hCD20 | 2F2 | mTNFRY |

**Table 11:** $EC_{50}$ values of mAb and mAb$^2$ molecules in the T cell activation assay.

| Test antibody molecule (Fcab/Fab target) | CD20 cells | $EC_{50}$ (nM) |
|---|---|---|
| none/mTNFRY | absent | 3.251 |
| none/mTNFRY | present | 2.516 |
| mTNFRY/hCD20 | present | 0.08299 |

**Table 12:** Details of antibody molecules tested

| Test antibody molecule (Fcab/Fab target) | Molecule type | Fab target | Fab clone | Fcab target |
|---|---|---|---|---|
| none/FITC | mAb | FITC | 4420 | none |
| mTNFRX/FITC | mAb$^2$ | FITC | 4420 | mTNFRX |
| none/EphA2 | mAb | EphA2 | E2A | none |
| mTNFRX/EphA2 | mAb$^2$ | EphA2 | E2A | mTNFRX |
| none/mTNFRX | mAb | mTNFRX | OX86 | none |

**Table 13:** $EC_{50}$ and maximum response values of mAb and mAb$^2$ molecules in the T cell activation assay.

| Antibody molecule + crosslinking agent (in the presence of HPAC cells) | $EC_{50}$ (nM) | $EC_{50}$ 95% Conf. Int. | Max response (mIL-2 pg/ml) | Max response 95% Conf. Int. |
|---|---|---|---|---|
| none/FITC + FITCdex | 22.16 | 0.0001078 to 4554989 | 1449 | -165.4 to 3064 |
| mTNFRX/FITC + FITCdex | 0.5484 | 0.2053 to 1.465 | 18520 | 16257 to 20782 |
| none/EphA2 | 0.07089 | 0.0001227 to 40.95 | 982.4 | 414.2 to 1551 |
| mTNFRX/FITC + none/EphA2 | | | | |
| mTNFRX/EphA2 | ~ 1.845e-005 | (Very wide) | 11030 | 9159 to 12901 |
| none/TNFRX + a-hFc | 0.8106 | 0.4725 to 1.39 | 17911 | 16515 to 19308 |
| none/FITC + FITCdex | 22.16 | 0.0001078 to 4554989 | 1449 | -165.4 to 3064 |

**Table 14:** Details of antibody molecules tested.

| Test antibody molecule (Fcab/Fab target) | Molecule type | Fab target | Fab clone | Fcab target |
|---|---|---|---|---|
| none/FITC | mAb | FITC | 4420 | none |
| mTNFRX/FITC | mAb$^2$ | FITC | 4420 | mTNFRX |
| none/VEGF | mAb | VEGF | R84 | none |
| mTNFRX/VEGF | mAb$^2$ | VEGF | R84 | mTNFRX |
| none/mTNFRX | mAb | mTNFRX | OX86 | none |

**Table 15:** Human IL-2 produced in response to mAb and mAb$^2$ molecules in the T cell activation assay.

| mIL-2 (pg/ml) | Average | | | | Standard deviation | | | |
|---|---|---|---|---|---|---|---|---|
| | none | a-hFc | VEGF | FITCdex | none | a-hFc | VEGF | FITCdex |
| none/FITC | -725.595 | 151.475 | 1867.88 | 161.805 | 242.5447 | 47.39737 | 845.629 | 20.61216 |
| none/VEGF | 46.175 | 1237.26 5 | 1907.74 5 | 970.845 | 438.4416 | 502.364 | 402.6761 | 134.0038 |
| none/mTNFR X | 296.835 | 7544.23 5 | 2773.35 | 2341.11 | 438.4416 | 3658.761 | 306.0358 | 1638.918 |
| mTNFRX/FIT C | 250.66 | 4742.62 5 | 2391.8 | 30161.8 1 | 783.6016 | 682.4641 | 233.5574 | 5421.84 |
| mTNFRx/VEG F | 6866.75 5 | 7343.16 5 | 21087.7 | 7953.35 5 | 3498.21988 1 | 2350.69871 2 | 2899.29336 6 | 1473.99944 1 |

**Table 16:** EC$_{50}$ and maximum response values of mAb and mAb$^2$ molecules in the T cell activation assay.

| Antibody molecule + crosslinking agent | EC$_{50}$ (nM) | EC$_{50}$ 95% Conf. Int. | Max response (mIL-2 pg/ml) | Max response 95% Conf. Int. |
|---|---|---|---|---|
| none/FITC + FITCdex | ~ 0.0003086 | Not determined | 244 | Not determined |
| none/VEGF + h-Fc | ~ 4.062e-005 | Not determined | 1294 | Not determined |
| mTNFRX/FITC + FITCdex | 1.15 | 0.4749 to 2.985 | 33698 | 27301 to 41962 |
| mTNFRX/VEGF | ~ 1528 | Not determined | ~ 900486 | (Very wide) |
| mTNFRX/VEGF + VEGF | 0.3623 | 0.1253 to 1.041 | 23043 | 18245 to 28541 |

Sequence Listing

Amino acid sequence of "wild-type" Fcab CH2 and CH3 domains without LALA mutation (SEQ ID NO: 1).

APELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTVD
KSRWQQGNVFSCSVMHEALHNHYTQKSLSLSPG

Amino acid sequence of "wild-type" Fcab CH2 and CH3 domains, comprising LALA mutation (underlined) (SEQ ID NO: 2)

APE<u>AA</u>GGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTVD
KSRWQQGNVFSCSVMHEALHNHYTQKSLSLSPG

Amino acid sequence of "wild-type" Fcab CH3 domain (SEQ ID NO: 3)

GQPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPG

The following numbered clauses, describing aspects of the invention, are part of the description.

1. An antibody molecule, which binds to a first antigen and a second antigen, the antibody molecule comprising:

(i) a CDR-based antigen-binding site for the first antigen; and
(ii) an antigen-binding site for the second antigen located in a constant domain of the antibody molecule, wherein said antigen-binding site comprises one or more amino acid modifications in one or more structural loops of the constant domain; and

wherein one of the first and second antigens is a tumour antigen and the other is a tumour necrosis factor receptor superfamily (TNFRSF) receptor on the surface of an immune cell.

2. The antibody molecule according to clause 1 wherein the antibody molecule is capable of activating the TNFRSF receptor on the immune cell in the presence of the tumour antigen.

3. The antibody molecule according to clause 1 or clause 2 wherein binding of the antibody molecule to the TNFRSF receptor and the tumour antigen causes clustering of the TNFRSF receptor on the immune cell.

4. The antibody molecule according to any one of the preceding clauses wherein the first antigen is a tumour antigen and the second antigen is a TNFRSF receptor on the surface of an immune cell.

5. The antibody molecule according to any one of the preceding clauses wherein the first antigen is a TNFRSF receptor on the surface of an immune cell and the second antigen is a tumour antigen.

6. The antibody molecule according to any one of the preceding clauses, wherein the TNFRSF receptor expressed on the immune cell requires clustering of three or more receptors for activation.

7. The antibody molecule according to any one of the preceding clauses, wherein the TNFRSF receptor is selected from the group consisting of CD27, EDA2R, EDAR, FAS, LTBR, RELT, TNFRSF1A, TNFRSF1B, TNFRSF6B, TNFRSF8, TNFRSF10A-10D, TNFRSF11A, TNFRSF11B, TNFRSF12A, TNFRSF13B, TNFRSF13C, TNFRSF14, TNFRSF17, TNFRSF18, TNFRSF19, TNFRSF21 and TNFRSF25.

8. The antibody molecule according to any one of the preceding clauses, wherein the immune cell is a T cell.

9. The antibody molecule according to any one of the preceding clauses, wherein the tumour antigen is a cell surface antigen on a cancer cell.

10. The antibody molecule according to clause 9, wherein the tumour antigen is a Tumour Associated Antigen (TAA).

11. The antibody molecule according to clause 10, wherein the Tumour Associated Antigen (TAA) is a MHC/peptide neoantigen or a tumour microenvironment antigen.

12. The antibody molecule according to clause 10, wherein the TAA is selected from the group consisting of HER2, FAP, EpCAM, CEACAM5, CD20, CD73, PSMA, mesothelin, EphA2, IGF1R, CD200, $\alpha_v\beta_6$, PDL1, B7H3 or EGFR.

13. The antibody molecule according to any one of clauses 1 to 8, wherein the tumour antigen is a soluble multimer.

14. The antibody molecule according to clause 13, wherein soluble multimer is at least a dimer, such as vascular endothelial growth factor (VEGF).

15. The antibody molecule according to clause 14, wherein soluble multimer is at least a trimer.

16. The antibody molecule according to any one of the preceding clauses wherein the antibody molecule is a whole antibody.

17. The antibody molecule according to clause 16 wherein the antibody molecule is an IgG.

18. The antibody molecule according to any one of the preceding clauses wherein the antibody molecule does not bind to Fc $\gamma$ receptors.

19. The antibody molecule according to any one of the preceding clauses wherein the antibody molecule has been modified to reduce or abrogate binding of the CH2 domain of the antibody molecule to one or more Fc $\gamma$ receptors.

20. The antibody molecule according to clause 19, wherein the Fc $\gamma$ receptor is selected from the group consisting of: Fc$\gamma$RI, Fc$\gamma$RIIa, Fc$\gamma$RIIb and Fc$\gamma$RIII.

21. The antibody molecule according to any one of the preceding clauses wherein the antibody molecule has one or more antigen-binding sites for the second antigen in the constant region.

22. The antibody molecule according to any one of the preceding clauses, wherein the constant domain is selected from the group consisting of CH3, CH2, CH1 and CL.

23. The antibody molecule according to clause 22, wherein the constant domain is a CH3 domain or a CH2 domain.

24. The antibody molecule according to clause 23 wherein the constant domain is a CH3 domain.

25. The antibody molecule according to clause 23, wherein the one or more structural loops are selected from the AB, CD and EF loops.

26. The antibody molecule according to any one of the preceding clauses, wherein the amino acid modification is an amino acid substitution, addition, or deletion.

27. The antibody molecule according to any one of the preceding clauses, wherein the antigen-binding site for the second antigen comprises one or more amino acid modifications in two or more structural loops of the constant domain.

28. The antibody molecule according to any one of the preceding clauses, wherein the CDR-based antigen binding site for the first antigen comprises three VH domain CDRs and three VL domain CDRs.

29. The antibody molecule according to clause 28 where the CDR-based antigen binding site is formed by an immunoglobulin VH domain and an immunoglobulin VL domain.

30. The antibody molecule according to clause 28 or 29 where the CDR-based antigen binding site for the first antigen is located in a variable region of the antibody molecule.

31. The antibody molecule according to any one of the preceding clauses wherein the antibody molecule has two CDR-based antigen binding domains for the first antigen.

32. A nucleic acid encoding an antibody molecule according to any one of clauses 1 to 31.

33. A vector comprising the nucleic acid of clause 32.

34. A recombinant host cell comprising the nucleic acid of clause 32, or the vector of clause 33.

35. A method of producing an antibody molecule according to any one of clauses 1 to 31 comprising culturing the recombinant host cell of clause 34 under conditions for production of the antibody molecule.

36. The method of clause 35 further comprising isolating and/or purifying the antibody molecule.

37. A pharmaceutical composition comprising an antibody molecule according to any one of clauses 1 to 31 and a pharmaceutically acceptable excipient.

38. An antibody molecule according to any of clauses 1 to 31 for use as a medicament.

39. An antibody molecule according to any one of clauses 1 to 31, for use in a method of treating cancer in a patient.

40. Use of an antibody molecule according to any one of clauses 1 to 31 in the manufacture of a medicament for use in a method of treating cancer in a patient.

41. A method of treating cancer in a patient, wherein the method comprises administering to the patient a therapeutically effective amount of an antibody molecule according to any one of clauses 1 to 31.

42. An antibody for use, use or method of treating according to any one of clauses 39 to 41, wherein the method further comprises administering at least one additional therapeutic agent to the patient.

**Claims**

1. An antibody molecule, which binds to a first antigen and a second antigen, the antibody molecule comprising:

   (i) a CDR-based antigen-binding site for the first antigen; and
   (ii) an antigen-binding site for the second antigen located in a constant domain of the antibody molecule, wherein said antigen-binding site comprises one or more amino acid modifications in one or more structural loops of the constant domain; and

   wherein one of the first and second antigens is a tumour antigen and the other is a tumour necrosis factor receptor superfamily (TNFRSF) receptor on the surface of an immune cell.

2. The antibody molecule according to claim 1, wherein the antibody molecule is capable of activating the TNFRSF receptor on the immune cell in the presence of the tumour antigen, and optionally wherein binding of the antibody molecule to the TNFRSF receptor and the tumour antigen causes clustering of the TNFRSF receptor on the immune cell.

3. The antibody molecule according to any one of the preceding claims wherein:

   (a) the first antigen is a tumour antigen and the second antigen is a TNFRSF receptor on the surface of an immune cell; or
   (b) the first antigen is a TNFRSF receptor on the surface of an immune cell and the second antigen is a tumour antigen.

4. The antibody molecule according to any one of the preceding claims, wherein:

   (a) the TNFRSF receptor expressed on the immune cell requires clustering of three or more receptors for activation; and/or
   (b) the TNFRSF receptor is selected from the group consisting of CD27, CD40, EDA2R, EDAR, FAS, LTBR, RELT, TNFRSF1A, TNFRSF1B, TNFRSF4, TNFRSF6B, TNFRSF8, TNFRSF9, TNFRSF10A-10D, TNFRSF11A, TNFRSF11B, TNFRSF12A, TNFRSF13B, TNFRSF13C, TNFRSF14, TNFRSF17, TNFRSF18, TNFRSF19, TNFRSF21 and TNFRSF25; and/or
   (c) the immune cell is a T cell; and/or
   (d) the tumour antigen is a cell surface antigen on a cancer cell, optionally

   wherein the tumour antigen is a Tumour Associated Antigen (TAA), optionally wherein the TAA (i) is a MHC/peptide neoantigen or a tumour microenvironment antigen, or (ii) is selected from the group consisting of HER2, FAP, EpCAM, CEACAM5, CD20, CD73, PSMA, mesothelin, EphA2, IGF1R, CD200, $\alpha_v\beta_6$, PDL1, B7H3 or EGFR.

5. The antibody molecule according to any preceding claim, wherein:

   (a) the tumour antigen is a soluble multimer, optionally wherein the soluble multimer is at least a dimer, such as vascular endothelial growth factor (VEGF), optionally wherein the soluble multimer is at least a trimer; and/or
   (b) the antibody molecule is a whole antibody, optionally wherein the antibody molecule is an IgG; and/or
   (c) the antibody molecule does not bind to Fc $\gamma$ receptors, optionally wherein the antibody molecule has been modified to reduce or abrogate binding of the CH2 domain of the antibody molecule to one or more Fc $\gamma$ receptors, optionally wherein the Fc $\gamma$ receptor is selected from the group consisting of: Fc$\gamma$RI, Fc$\gamma$RIIa, Fc$\gamma$RIIb and Fc$\gamma$RIII.

6. The antibody molecule according to any one of the preceding claims, wherein the constant domain is selected from the group consisting of CH3, CH2, CH1 and CL, optionally wherein the constant domain is a CH3 domain or a CH2 domain, optionally wherein the constant domain is a CH3 domain, and/or wherein the one or more structural loops are selected from the AB, CD and EF loops.

7. The antibody molecule according to any one of the preceding claims, wherein:

   (a) the amino acid modification is an amino acid substitution, addition, or deletion; and/or
   (b) the antigen-binding site for the second antigen comprises one or more amino acid modifications in two or more structural loops of the constant domain; and/or

(c) the CDR-based antigen binding site for the first antigen comprises three VH domain CDRs and three VL domain CDRs, optionally wherein the CDR-based antigen binding site is formed by an immunoglobulin VH domain and an immunoglobulin VL domain; and/or

(d) the antibody molecule has two CDR-based antigen binding sites for the first antigen.

8. A nucleic acid encoding an antibody molecule according to any one of claims 1 to 7.

9. A vector comprising the nucleic acid of claim 8.

10. A recombinant host cell comprising the nucleic acid of claim 8, or the vector of claim 9.

11. A method of producing an antibody molecule according to any one of claims 1 to 7 comprising culturing the recombinant host cell of claim 10 under conditions for production of the antibody molecule, optionally further comprising isolating and/or purifying the antibody molecule.

12. A pharmaceutical composition comprising an antibody molecule according to any one of claims 1 to 7 and a pharmaceutically acceptable excipient.

13. An antibody molecule according to any of claims 1 to 7 for use as a medicament.

14. An antibody molecule according to any one of claims 1 to 7, for use in a method of treating cancer in a patient.

15. An antibody for use according to claim 14 wherein the method further comprises administering at least one additional therapeutic agent to the patient.

Figure 1

Figure 2

Figure 3

Figure 4

Figure 5

A

B

Figure 6

A

B

Figure 7

## mTNFRx/EphA2 in CT26 model

Figure 8

**mTNFRY/EGFR in CT26.hEGFR**

Figure 9

Figure 10

mTNFRX/VEGF mAb$^2$ assay

Figure 11

Figure 12

Europäisches
Patentamt

European
Patent Office

Office européen
des brevets

**EUROPEAN SEARCH REPORT**

Application Number

EP 22 18 2924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 546 268 A1 (STAR BIOTECHNOLOGISCHE FORSCHUNGS UND ENTWICKLUNGSGES M B H F [AT]) 16 January 2013 (2013-01-16) * page 5, paragraph 47 – paragraph 51; claims 1-3 * * paragraph [0059] * * paragraph [0063] * * paragraph [0047] * * example 3 * | 1-15 | INV. C07K16/22 C07K16/28 C07K16/30 |
| A | XU DANLIN ET AL: "In vitro characterization of five humanized OKT3 effector function variant antibodies", CELLULAR IMMUNOLOGY, ACADEMIC PRESS, SAN DIEGO, CA, US, vol. 200, no. 1, 25 February 2000 (2000-02-25), pages 16-26, XP002376698, ISSN: 0008-8749, DOI: 10.1006/CIMM.2000.1617 * Discussion on pages 22 and 23 * | 1 | |
| A | US 2012/276104 A1 (WOISETSCHLAGER MAX [AT]) 1 November 2012 (2012-11-01) * claim 1 * * page 18, column 19; example 1 * * page 19; example 3 * | 1 | TECHNICAL FIELDS SEARCHED (IPC) C07K A61K |
| A | WO 2016/177802 A1 (PIERIS PHARMACEUTICALS GMBH [DE]) 10 November 2016 (2016-11-10) * page 36, paragraph 176; claim 1 * * page 39, paragraph 189 * * page 3, paragraphs 9, 10 * * figure 1 * * paragraph [0094] – paragraph [0095] * | 1 | |

-/--

The present search report has been drawn up for all claims

| Place of search | Date of completion of the search | Examiner |
|---|---|---|
| The Hague | 30 November 2022 | Sitch, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 22 18 2924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALENA JANDA ET AL: "Ig Constant Region Effects on Variable Region Structure and Function", FRONTIERS IN MICROBIOLOGY, vol. 7, 4 February 2016 (2016-02-04), XP55436953, DOI: 10.3389/fmicb.2016.00022 * abstract * | 1 | |
| A | CARTER PAUL ET AL: "IDENTIFICATION AND VALIDATION OF CELL SURFACE ANTIGENS FOR ANTIBODY TARGETING IN ONCOLOGY", ENDOCRINE RELATED CANCER, BIOSCIENTIFICA LTD, GB, vol. 11, no. 4, 1 January 2004 (2004-01-01), pages 659-687, XP009078100, ISSN: 1351-0088, DOI: 10.1677/ERC.1.00766 * Page 64, paragraph before 'Source material * | 1 | |
| A | Kim Et Al: "A Perspective on the Effects of Antigen Shedding on Targeted Deliveryof Immunotoxins in Solid Tumors: A Mathematical Model Study", Bulletin of the Korean Chemical Society, 1 January 2016 (2016-01-01), pages 1977-1984, XP55931325, Retrieved from the Internet: URL:https://onlinelibrary.wiley.com/doi/full/10.1002/bkcs.11007 [retrieved on 2022-06-14] * abstract * | 1 | **TECHNICAL FIELDS SEARCHED (IPC)** |

The present search report has been drawn up for all claims

| Place of search | Date of completion of the search | Examiner |
|---|---|---|
| The Hague | 30 November 2022 | Sitch, David |

## ANNEX TO THE EUROPEAN SEARCH REPORT
## ON EUROPEAN PATENT APPLICATION NO.

EP 22 18 2924

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2022

| Patent document cited in search report | | Publication date | Patent family member(s) | | Publication date |
|---|---|---|---|---|---|
| EP 2546268 | A1 | 16-01-2013 | NONE | | |
| US 2012276104 | A1 | 01-11-2012 | EP | 2407487 A1 | 18-01-2012 |
| | | | US | 2012276104 A1 | 01-11-2012 |
| | | | WO | 2012007167 A1 | 19-01-2012 |
| WO 2016177802 | A1 | 10-11-2016 | AU | 2016258977 A1 | 30-11-2017 |
| | | | BR | 112017020434 A2 | 26-06-2018 |
| | | | CA | 2980840 A1 | 10-11-2016 |
| | | | CN | 107636014 A | 26-01-2018 |
| | | | CN | 114316067 A | 12-04-2022 |
| | | | EP | 3292148 A1 | 14-03-2018 |
| | | | HK | 1249526 A1 | 02-11-2018 |
| | | | JP | 6783797 B2 | 11-11-2020 |
| | | | JP | 2018515085 A | 14-06-2018 |
| | | | KR | 20170138574 A | 15-12-2017 |
| | | | RU | 2017135596 A | 04-06-2019 |
| | | | SG | 11201708334R A | 29-11-2017 |
| | | | US | 2019010248 A1 | 10-01-2019 |
| | | | US | 2021198380 A1 | 01-07-2021 |
| | | | WO | 2016177802 A1 | 10-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016177802 A1
- EP 0120694 A
- EP 0125023 A
- EP 184187 A
- GB 2188638 A
- EP 239400 A
- WO 2006072620 A
- WO 2009132876 A
- WO 0020581 A
- WO 1992020356 A1
- WO 1994005304 A1

- WO 1994023031 A1
- WO 1995020974 A1
- WO 1995023874 A1
- WO 1996026214 A1
- US 8529902 B2
- US 8637017 B2
- US 6217866 B1
- WO 2004014292 A2
- US 20090175791 A1
- US 8461308 B2
- US 8771690 B2

**Non-patent literature cited in the description**

- **WAJANT.** *Cell Death Differ.,* 2015, vol. 22 (1), 1727-41
- **HINNER et al.** Costimulatory T-cell engagement by PRS-343, a CD137 (4-1BB)/HER2 bispecific, leads to tumour growth inhibition and TIL expansion in humanized mouse model. *CRI-CIMT-AACR International Cancer Immunotherapy Conference: Translating Science into Survival,* 25 September 2016
- **HU et al.** *Cancer Res.,* 1996, vol. 56 (13), 3055-61
- **HOLLIGER ; HUDSON.** *Nature Biotechnology,* 2005, vol. 23 (9), 1126-1136
- **BRUHNS et al.** *Blood,* 2009, vol. 113 (16), 3716-25
- **XU et al.** *Cellular Immunology,* 2000, vol. 200 (1), 16-26
- **SEGAL et al.** *PNAS,* 1974, vol. 71, 4298-4302
- **AMIT et al.** *Science,* 1986, vol. 233, 747-753
- **CHOTHIA et al.** *J. Mol. Biol.,* 1987, vol. 196, 901-917
- **CHOTHIA et al.** *Nature,* 1989, vol. 342, 877-883
- **CATON et al.** *J. Immunol.,* 1990, vol. 144, 1965-1968
- **SHARON et al.** *PNAS,* 1990, vol. 87, 4814-4817
- **SHARON et al.** *J. Immunol.,* 1990, vol. 144, 4863-4869
- **KABAT et al.** *J. Immunol.,* 1991, vol. 147, 1709-1719
- **KABAT, E.A et al.** Sequences of Proteins of Immunological Interest. U.S.Department of Health and Human Services, 1991

- **LEFRANC, M.-P. et al.** *Nucleic Acids Res.,* 2015, vol. 43, D413-22
- **WOZNIAK-KNOPP G et al.** *Protein Eng Des.,* 2010, vol. 23 (4), 289-297
- **CHEN ; MELLMAN.** *Immunity,* 2013, vol. 39 (1), 1-10
- **SIMPSON et al.** *Nature Rev,* 2005, vol. 5, 615-625
- **GURE et al.** *Clin Cancer Res,* 2005, vol. 11, 8055-8062
- **VELAZQUEZ et al.** *Cancer Immun,* 2007, vol. 7, 1-1
- **ANDRADE et al.** *Cancer Immun,* 2008, vol. 8, 2
- **TINGUELY et al.** *Cancer Science,* 2008
- **NAPOLETANO et al.** *Am J of Obstet Gyn,* 2008, vol. 198 (99), 91-97
- **CARTER et al.** *Endocr Relat Cancer,* 2004, vol. 11 (4), 659-87
- **SCOTT, A. M. ; RENNER, C.** *Tumour Antigens Recognized by Antibodies,* 2001
- **ELS ; CHEEVER et al.** *Clin Cancer Res,* 2009, vol. 15 (17), 5323-37
- **MALARKANNAN et al.** *Immunity,* June 1999, vol. 10 (6), 681-90
- **GUBIN et al.** *J Clin Invest.,* 2015, vol. 125 (9), 3413-21
- Cancer Vaccines and Immunotherapy. Cambridge University Press, 2000
- **BHOME et al.** *Front Cell Dev Biol.,* 2015, vol. 3, 33
- **RUSSELL et al.** Molecular Cloning: a Laboratory Manual. Cold Spring Harbor Laboratory Press, 2001

- Current Protocols in Molecular Biology. John Wiley & Sons, 1992
- Harrison's Principles of Internal Medicine. McGraw-Hill, 2001
- **LEDERMANN et al.** *Int. J. Cancer,* 1991, vol. 47, 659-664
- **BAGSHAWE et al.** *Antibody, Immunoconjugates and Radiopharmaceutical,* 1991, vol. 4, 915-922
- *Physician's Desk Reference,* 2003
- **ROSENBERG, S.** *Development of Cancer Vaccines,* 2000
- *J. Immunol.,* 01 April 2004, vol. 172 (7), 4618-23
- **VORON T ; COLUSSI O ; MARCHETEAU E et al.** VEGF-A modulates expression of inhibitory checkpoints on CD8+ T cells in tumors. *The Journal of Experimental Medicine,* 2015, vol. 212 (2), 139-148
- **BEDZYK, W. D. et al.** *J. Biol. Chem,* 1989, vol. 264, 1565-1569
- **FARR A et al.** *J. Histochem. Cytochem.,* 1991, vol. 39, 645
- **YAMASHITA et al.** *Eur J Immunol.,* 1998, vol. 28 (10), 2981-90